# EUROPEAN PATENT SPECIFICATION

(11) **EP 2 253 710 B1**
(45) Date of publication and mention of the grant of the patent: **07.12.2016**
(21) Application number: 09710722.1
(22) Date of filing: 16.02.2009
(51) Int. Cl.: C12P 7/64, C12P 7/62, C07C 68/06, C07C 69/96

(54) **PROCESS FOR PREPARING ASYMMETRICAL CARBONATE COMPOUNDS**
VERFAHREN ZUR HERSTELLUNG ASYMMETRISCHER CARBONATVERBINDUNGEN
PROCÉDÉ POUR LA PRÉPARATION DE COMPOSÉS DE CARBONATE ASYMMÉTRIQUES

(30) Priority: 14.02.2008 JP 2008032509; 27.03.2008 JP 2008084171
(43) Date of publication of application: 24.11.2010
(73) Proprietor: Ube Industries, Ltd., Ube-shi, Yamaguchi 755-8633 (JP)
(72) Inventor: YAMAMOTO, Yasuhito, Yamaguchi 755-8633 (JP); OKADA, Naoko, Yamaguchi 755-8633 (JP); HIROTSU, Kenji, Yamaguchi 755-8633 (JP); MIYATA, Hiroyuki, Yamaguchi 755-8633 (JP); YAMAMOTO, Yasushi, Yamaguchi 755-8633 (JP)
(74) Representative: Hoffmann Eitle
(86) International application number: PCT/JP2009/052586
(87) International publication number: WO 2009/102069

(56) References cited:
- EP-A1- 0 885 874
- DE-A1- 4 226 043
- JP-A- 9 270 270
- JP-A- 2000 281 630
- JP-A- 2000 344 718
- JP-A- 2001 002 624
- US-A- 4 332 744
- US-A1- 2002 102 671
- POHLMAN M. ET AL.: 'Allylic alkylation versus Michael induced ring closure: chelated enolates as versatile nucleophiles.' JOURNAL OF ORGANIC CHEMISTRY vol. 69, no. 20, 2004, pages 6909 - 6912, XP008140697
- MANDAI T. ET AL.: 'Tandem Pd-catalyzed carbonylation and intramolecular ene reaction of 1-(2-methoxycarbonylethynyl)-4-alkenyl methyl carbonates.' TETRAHEDRON LETTERS vol. 35, no. 31, 1994, pages 5701 - 5704, XP008140698
- HACKING M. ET AL.: 'Lipase catalyzed reactions of aliphatic and arylaliphatic carbonic acid esters.' JOURNAL OF MOLECULAR CATALYSIS B: ENZYMATIC vol. 9, no. 4-6, 2000, pages 201 - 208, XP009114319

## Description

### TECHNICAL FIELD

The present invention relates to a process for preparing an asymmetric carbonate compound by reacting a symmetric carbonate compound and an ester compound.

### BACKGROUND ART

An asymmetric carbonate compound is useful, for example, as a perfume, a non-aqueous organic electrolyte, a solvent and various kinds of organic synthetic reagents, etc. (for example, see Patent Literatures 1 to 5).

As a method for obtaining an asymmetric carbonate compound, it has heretofore been known a disproportionation transesterfication reaction of two kinds of symmetric carbonate compounds in the presence of a heterogeneous catalyst (for example, see Patent Literatures 4 and 5). However, in Patent Literature 4, for example, the reaction is carried out at high temperature for extremely long time, but its yield is relatively low as 48.8%, so that it cannot be said that it is an industrially satisfied preparation process.

Also, an asymmetric carbonate compound can be synthesized by using an ester compound as a starting material. For example, it has been known a method in which a cyclic ester compound and an alcohol compound are reacted in the presence of an acid or a base, and the resulting hydroxyalkanoic acid ester is reacted with a chloroformate in the presence of a tertiary amine to obtain an asymmetric carbonate compound (for example, see Patent Literature 1 and non-Patent Literature 1). However, this method requires many reaction steps, and involves problems on the operation and safety since it uses a haloformate compound such as a chloroformate, etc., which has toxicity.

On the other hand, among lipases which have been generally known as a hydrolase, *Candida Antarctica* lipase B has been used for synthesizing a β-keto ester compound which has been transesterified from a β-keto ester compound and an alcohol compound (for example, see Patent Literature 6).

M. Hacking et al.: "Lipase catalyzed reactions of aliphatic and arylaliphatic carbonic acid esters.", Journal of Molecular Catalysis B: Enzymatic, vol. 9, no. 4-6, 2000, pages 201-208, teaches a method for producing an asymmetrical carbonate by reacting a symmetrical carbonate and an alcohol in the presence of a lipase derived from Candida Antarctica.

However, the reaction of a cyclic ester or a lower aliphatic acid ester such as an acetate compound and a symmetric carbonate compound proceeds in the presence of a Hydrolase catalyst. As of today, there is no report that the target asymmetric carbonate compound can be obtained by a process according to claim 1.

[Patent Literature 1] JP S61-93141A
[Patent Literature 2] JP H10-338663A
[Patent Literature 3] JP 2007-268420A
[Patent Literature 4] JP 2000-344718A
[Patent Literature 5] JP 2001-2624A
[Patent Literature 6] US Patent No. 6,642,035
[Non-Patent Literature 1] J. Org. Chem., 1988, Vol. 53(5), 1064-1071

### DISCLOSURE OF THE INVENTION

### PROBLEMS TO BE SOLVED BY THE INTENTION

An object of the present invention is to provide a process for preparing an symmetric carbonate compound from a symmetric carbonate compound and an ester compound with good yield, which is also simple and easy, and industrially suitable.

### MEANS TO SOLVE THE PROBLEMS

The present inventors have found that a target asymmetric carbonate compound can be obtained with good yield by reacting a symmetric carbonate compound and an ester compound in the presence of a hydrolase catalyst, which is also simple and easy, and industrially suitable, whereby the present invention has accomplished.

Embodiment 1 of the present invention relates to a process for preparing an asymmetric carbonate compound which comprises allowing a symmetric carbonate compound represented by the formula (1): 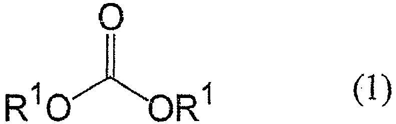 (wherein R¹s are the same, and represent monovalent hydrocarbon groups which may have a substituent(s))
to react with an ester compound represented by the formula (2): 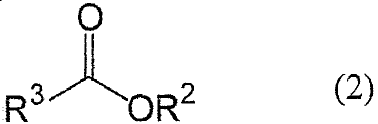 [wherein
R² and R³ are each independently, and represent a monovalent hydrocarbon group which may have a substituent(s) (here, the hydrocarbon group of R² and R³ may be interrupted by a bonding group selected from the group consisting of an ether bond, a thioether bond, an amide bond, a carbonyl bond and an ester bond), or
R² and R³ are bonded to each other to form Z, and Z represents a divalent hydrocarbon group which may have a substituent(s) (here, the hydrocarbon group of Z may be interrupted by a bonding group selected from the group consisting of an ether bond, a thioether bond, an amide bond, a carbonyl bond and an ester bond),
provided that R² is not the same as R¹ of the formula (1), and the number of ester bonds contained in the formula (2a) is 2 or less in total] in the presence of a hydrolase, to obtain an asymmetric carbonate compound represented by the formula (3): 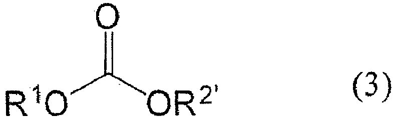 (wherein
R¹ is as defined above, and
R^{2'} is R²,
provided that when R² and R³ are bonded together to form Z, R^{2'} is -Z-C(=O)-O-R¹)).

Embodiment 2 of the present invention relates to the process for preparing an asymmetric carbonate compound of embodiment 1, wherein the symmetric carbonate compound represented by the formula (1): 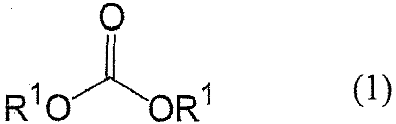 (wherein R¹s are the same, and represent monovalent hydrocarbon groups which may have a substituent(s)) and an ester compound represented by the formula (2a): 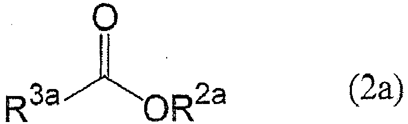 [wherein
R^{2a} and R^{3a} are each independently, and represent a monovalent hydrocarbon group which may have a substituent(s) (here, the hydrocarbon group of R^{2a} and R^{3a} may be interrupted by a bonding group selected from the group consisting of an ether bond, a thioether bond, an amide bond, a carbonyl bond and an ester bond),
provided that R^{2a} is not the same as R¹ of the formule (1), and the number of ester bonds contained in the formula (2a) is made 2 or less in total] are reacted in the presence of a hydrolase, to obtain an asymmetric carbonate compound represented by the formula (3a): 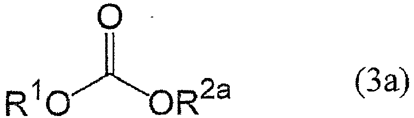 (wherein R¹ and R^{2a} are the same as defined above).

Embodiment 3 of the present invention relates to the process for preparing an asymmetric carbonate compound of embodiment 2, wherein the reaction of the symmetric carbonate compound represented by the formula (1) and the ester compound represented by the formula (2a) is carried out while removing a transesterificated compound represented by the formula (4a): 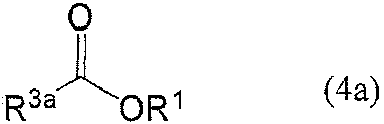 (wherein R¹ and R^{3a} are as defined in the present invention 2) which is by-produced by the reaction.

Embodiment 4 of the present invention relates to the process for preparing an asymmetric carbonate compound of embodiment 3, wherein removal of the transesterificated compound is carried out at a reaction pressure of 0.13 kPa or more and less than 101.3 kPa.

Embodiment 5 of the present invention relates to the process for preparing an asymmetric carbonate compound of any one of embodiments 2 to 4, wherein R¹ and R^{2a} are different from each other, and each represents a linear alkyl group having 1 to 6 carbon atoms, a branched alkyl group having 3 to 6 carbon atoms or a cycloalkyl group having 3 to 6 carbon atoms.

Embodiment 6 of the present invention relates to the process for preparing an asymmetric carbonate compound of any one of embodiments 2 to 5, wherein R¹ is methyl, ethyl, n-propyl, isopropyl, n-butyl, s-butyl, isobutyl, t-butyl or benzyl.

Embodiment 7 of the present invention relates to the process for preparing an asymmetric carbonate compound of any one of embodiments 2 to 6, wherein R¹ is methyl or ethyl.

Embodiment 8 of the present invention relates to the process for preparing an asymmetric carbonate compound of embodiment 1, wherein the symmetric carbonate compound represented by the formula (1): 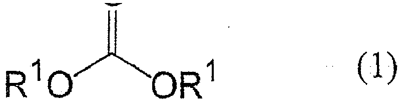 (wherein R¹s are the same, and represent monovalent hydrocarbon groups which may have a substituent(s))
and an ester compound represented by the formula (5a): 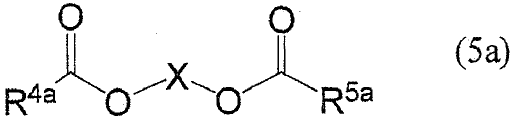 [wherein
R^{4a} and R^{5a} are each independently, and represent a monovalent hydrocarbon group which may have a substituent(s),
X represents a divalent hydrocarbon group which may have a substituent(s) (here, the hydrocarbon group may be interrupted by a bonding group selected from the group consisting of an ether bond, a thioether bond, an amide bond and a carbonyl bond)]
are reacted in the presence of a hydrolase, to obtain an asymmetric carbonate compound represented by the formula (6a): 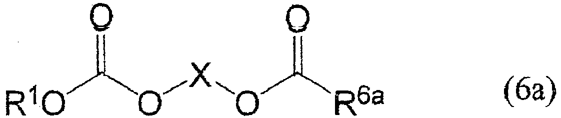 (wherein
R^{6a} is R^{4a} or R^{5a}, and
R¹, X, R^{4a} and R^{5a} are as defined above).

Embodiment 9 of the present invention relates to the process for preparing an asymmetric carbonate compound of embodiment 8, wherein R^{4a} and R^{5a} each independently repesents a linear alkyl group having 1 to 6 carbon atoms, a branched alkyl group having 3 to 6 carbon atoms or a cycloalkyl group having 3 to 6 carbon atoms, and X is an alkylene group having 1 to 6 carbon atoms or an alkenylene group having 1 to 6 carbon atoms, or a linear alkylene group having 2 to 6 carbon atoms which is interrupted by an ether bond.

Embodiment 10 of the present invention relates to the process for preparing an asymmetric carbonate compound of embodiment 1, wherein the symmetric carbonate compound represented by the formula (1): 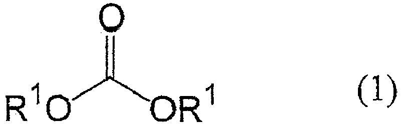 (wherein R¹s are the same, and represent monovalent hydrocarbon groups which may have a substituent(s))
and an ester compound represented by the formula (5a): 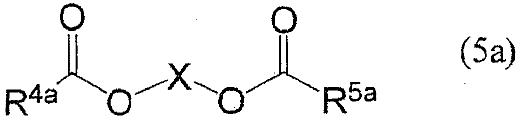 [wherein
R^{4a} and R^{5a} are each independently, and represent a monovalent hydrocarbon group which may have a substituent(s),
X represents a divalent hydrocarbon group which may have a substituent(s) (here, the hydrocarbon group may be interrupted by a bonding group selected from the group consisting of an ether bond, a thioether bond, an amide bond and a carbonyl bond)]
are reacted in the presence of a hydrolase, to obtain an asymmetric carbonate compound represented by the formula (7a): 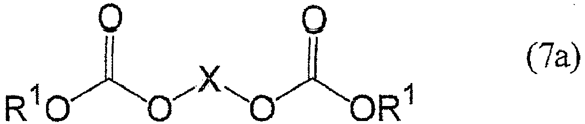 (wherein R¹ and X are the same as defined above).

Embodiment 11 of the present invention relates to the process for preparing an asymmetric carbanate compound of embodiment 1, wherein the symmetric carbonate compound represented by the formula (1): 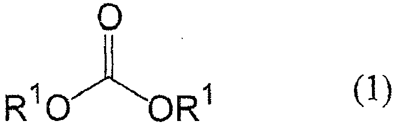 (wherein R¹s are the same, and represent monovalent hydrocarbon groups which may have a substituent(s))
and an ester compound represented by the formula (8a): 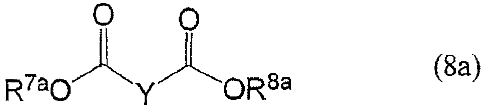 [wherein
R^{7a} and R^{8a} are each independently, and represent a monovalent hydrocarbon group which may have a substituent(s),
Y represents a divalent hydrocarbon group which may have a substituent(s) (here, a hydrocarbon group may be interrupted by a bonding group selected from the group consisting of an ether bond, a thioether bond, an amide bond and a carbonyl bond)]
are reacted in the presence of a hydrolase, to obtain an asymmetric carbonate compound represented by the formula (9a): 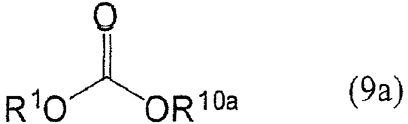 (wherein
R^{10a} is R^{7a} or R^{8a}, and
R¹, R^{7a} and R^{8a} are as defined above).

Embodiment 12 of the present invention relates to the process for preparing an asymmetric carbonate compound of embodiment 1, wherein the symmetric carbonate compound represented by the formula (1): 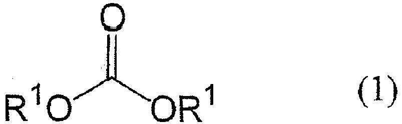 (wherein R¹s are the same, and represent monovalent hydrocarbon groups which may have a substituent(s))
and a cyclic ester compound represented by the formula (10): 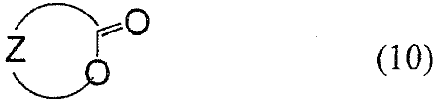 [wherein Z represents a divalent hydrocarbon group which may have a substituent(s) (here, a hydrocarbon group may be interrupted by a bonding group selected from the group consisting of an ether bond, a thioether bond, an amide bond, a carbonyl bond and an ester bond), provided that the number of ester bonds contained in the formula (10) is made 2 or less in total]
are reacted in the presence of a hydrolase, to obtain an asymmetric carbonate compound represented by the formula (11): 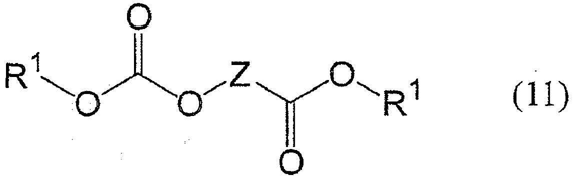 (wherein R¹ and Z are as defined above, R¹s in the formula (11) are the same with each other).

Embodiment 13 of the present invention relates to the process for preparing an asymmetric carbonate compound of embodiment 12, wherein the cyclic ester compound represented by the formula (10) is δ-valerolactone, β-propiolactone or ε-caprolactone.

Embodiment 14 of the present invention relates to the process for preparing an asymmetric carbonate compound of any one of embodiments 1 to 13, wherein the hydrolase is at least one hydrolase selected from the group consisting of a protease, an esterase and a lipase.

Embodiment 15 of the present invention relates to the process for preparing an asymmetric carbonate compound of embodiment 14, wherein the hydrolase is a lipase.

Embodiment 15 of the present invention relates to the process for preparing an asymmetric carbonate compound of embodiment 15, wherein the hydrolase is a lipase originated from *Candida Antarctica.*

Embodiment 17 of the present invention relates to the process for preparing an asymmetric carbonate compound of any one of embodiments 1 to 16, wherein an organic solvent is used.

Embodiment 18 of the present invention relates to the process for preparing an asymmetric carbonate compound of embodiment 17, wherein the organic solvent is at least one organic solvent selected from the group consisting of cyclohexane, toluene and t-butylmethyl ether.

Embodiment 19 of the present invention relates to the process for preparing an asymmetric carbonate compound of any one of embodiments 1 to 18, wherein an amount of the symmetric carbonate compound represented by the formula (1) is 0.1 to 100 mol based on 1 mol of the ester compound represented by the formula (2).

Embodiment 20 of the present invention relates to the process for preparing an asymmetric carbonate compound of any one of embodiments 1 to 19, wherein the reaction temperature is 0 to 130°C.

### EFFECTS OF THE INVENTION

According to the present invention, an asymmetric carbonate compound can be prepared from a symmetric carbonate compound represented by the formula (1) and an ester compound represented by the formula (2) with good yield, by the simple and easy, and industrially suitable process.

Also, in the preparation process of the asymmetric carbonate compound of the present invention, there is a case where the transesterificated compound represented by the formula (4a) which is by-produced when the ester compound represented by the formula (2a) is used as a starting material has a lower boiling point than those of the symmetric carbonate compound and ester compound which are starting materials, and the asymmetric carbonate compound which is a product. Thus, in the present invention, there is also found out a process for preparing a product while removing the by-producing transesterificated compound represented by the formula (4a) out of the reaction system. In the futher process comprising the removing step of the transesterificated compound represented by the formula (4a), the reaction proceeds rapidly, so that the target asymmetric carbonate compound can be obtained within a shorter reaction time.

According to the present invention, the asymmetric carbonate compound is produced by using a hydrolase, it can be provided a chemically safer product reduced any migration of impurities such as a metal salt or a halide, etc., which could occur in the product obtained by the conventional preparation method using an asymmetric carbonate compound. That is, when the asymmetric carbonate compound obtained by the preparation process of the present invention is used, for example, for a non-aqueous organic electrolyte or a solvent for producing electric and electronic materials, the lowering in electric characteristics accompanied by a halogen ion or a metal ion generating from the product can be restrained, so that the preparation process of the present invention is extremely excellent.

### BEST MODE TO CARRY OUT THE INVENTION

### Reaction scheme [I]

In the present invention, according to the reaction of the carbonate compound represented by the formula (1) and the ester compound represented by the formula (2) in the presence of a hydrolase, an asymmetric carbonate compound represented by the formula (3) is formed (Reaction scheme [I]). 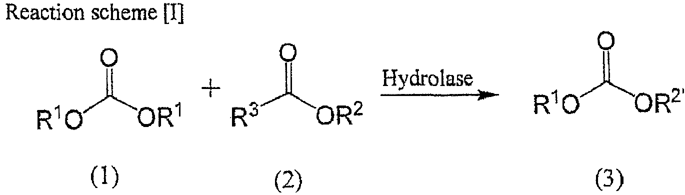 (wherein R¹, R², R^{2'} and R³ are the same as defined above.)

In Reaction scheme [I], R¹ represents a monovalent hydrocarbon group which may have a substituent(s). In the symmetric carbonate compound represented by the formula (1), R¹s are the same.

The hydrocarbon group of R¹ may be mentioned, for example, a linear alkyl group having 1 to 20 carbon atoms such as a methyl group, ethyl group, propyl group, butyl group, pentyl group, hexyl group, etc.; a branched alkyl group having 3 to 20 carbon atoms such as isopropyl group, isobutyl group, etc.; a cycloalkyl group having 3 to 20 carbon atoms such as cyclopropyl group, cyclobutyl group, cyclohexyl, etc.; an alkenyl group having 2 to 12 carbon atoms such as vinyl group, allyl group, isopropanyl group, etc.; an alkynyl group having 2 to 20 carbon atoms such as ethynyl group, propargyl group, etc.; an aralkyl group having 7 to 20 carbon atoms such as benzyl group, phenethyl group, etc.; or an aryl group having 6 to 20 carbon atoms such as phenyl group, naphthyl group and biphenyl group, etc. Incidentally, these groups contain various kinds of isomers.

As the hydrocarbon group of R¹, there may be preferably mentioned a linear alkyl group having 1 to 6 carbon atoms, a branched alkyl group having 3 to 6 carbon atoms, a cycloalkyl group having 3 to 6 carbon atoms, an aralkyl group having 7 to 20 carbon atoms and phenyl group; more preferably a linear alkyl group having 1 to 4 carbon atoms, a branched alkyl group having 3 to 6 carbon atoms, a cycloalkyl group having 3 to 6 carbon atoms, benzyl group and phenyl group; particularly preferably a methyl group, ethyl group, n-propyl group, isopropyl group, n-butyl group, t-butyl group, benzyl group, phenyl group. Incidentally, these groups contain various kinds of isomers.

The hydrocarbon group of R¹ may have a substituent(s), and as the substituent(s), there may be mentioned, for example, a halogen atom (fluorine atom, chlorine atom, bromine atom, iodine atom), an alkoxy group having 1 to 6 carbon atoms such as methoxy group, ethoxy group, propoxy group, butoxy group, etc., an alkylthio group having 1 to 6 carbon atoms, alkoxy group, a dialkylamino group which is disubstituted by alkyl groups having 1 to 6 carbon atoms such as dimethylamino group, diethylamino group, dipropylamino group, etc., cyano group, nitro group and acetyl group such as acyl group, etc. The substituent(s) may be either 1 or a plural number.

R¹ is preferably a linear alkyl group having 1 to 6 carbon atoms, a branched alkyl group having 3 to 6 carbon atoms, a cycloalkyl group having 3 to 6 carbon atoms, an aralkyl group having 7 to 20 carbon atoms, phenyl group; a linear alkyl group having 1 to 6 carbon atoms or a branched alkyl group having 3 to 6 carbon atoms each of which is substituted by a halogen atom, cyano group, nitro group, an alkoxy group having 1 to 6 carbon atoms or an alkylthio group having 1 to 6 carbon atoms including fluoromethyl group, difluoromethyl group, trifluoromethyl group, chloromethyl group, dichloromethyl group, trichloromethyl group, cyanomethyl group, nitromethyl group, fluoro-ethyl group, difluoroethyl group, trifluoroethyl group, perfluoroethyl group, chloroethyl group, dichloroethyl group, trichloroethyl group, cyanoethyl group, nitroethyl group, methoxyethyl group, ethoxyethyl group, t-butoxyethyl group, methylthioethyl group, ethylthioethyl group, etc.; a phenyl group which is substituted by a halogen atom, cyano group, nitro group, an alkoxy group having 1 to 6 carbon atoms or a dialkylamino group which is disubstituted by alkyl groups having 1 to 6 carbon atoms including fluorophenyl group, chlorophenyl group, bromophenyl group, iodophenyl group, methoxyphenyl group, dimethoxyphenyl group, ethoxyphenyl group, t-butoxyphenyl group, (dimethylamino)phenyl group, (diethylamino)phenyl group, nitrophenyl group, dinitrophenyl group, cyanophenyl group, etc.; an aralkyl group having 7 to 20 carbon atoms which is substituted by a halogen atom, cyano group, nitro group or an alkoxy group having 1 to 4 carbon atoms including fluorobenzyl group, chlorobenzyl group, bromobenzyl group, iodobenzyl group, methoxybenzyl group, dimethoxybenzyl group, nitrobenzyl group, dinitrobenzyl group and cyanobenzyl group, etc.

R¹ is more preferably a linear alkyl group having 1 to 6 carbon atoms, a branched alkyl group having 3 to 6 carbon atoms, a cycloalkyl group having 3 to 6 carbon atoms, benzyl group and phenyl group, fluoromethyl group, difluoromethyl group, trifluoromethyl group, cyanomethyl group, nitromethyl group; fluoroethyl group, trifluoroethyl group, trichloroethyl group, cyanoethyl group, nitroethyl group, methoxyethyl group, ethoxyethyl group, t-butoxyethyl group, fluorophenyl group, chlorophenyl group, bromophenyl group, iodophenyl group, ethoxyphenyl group, dimethoxyphenyl group, ethoxyphenyl group, t-butoxyphenyl group, nitrophenyl group, dinitrophenyl group, cyanophenyl group, fluorobenzyl group, chlorobenzyl group, bromobenzyl group, iodobenzyl group, methoxybenzyl group, dimethoxybenzyl group, nitrobenzyl group, dinitrobenzyl group and cyanobenzyl group; particularly preferably methyl group, ethyl group, n-propyl group, isopropyl group, n-butyl group, s-butyl group, isobutyl group, t-butyl group and benzyl group.

In Reaction scheme [I], R² and R³ are each independently, and represent a monovalent hydrocarbon group which may have a substituent(s) (here, a hydrocarbon group of R² and R³ may be interrupted by a bonding group selected from the group consisting of an ether bond, a thioether bond, an amide bond, a carbonyl bond and an ester bond), or R² and R³ are bonded to each other to form Z, and Z is a divalent hydrocarbon group (here, a hydrocarbon group of Z may be interrupted by a bonding group selected from the group consisting of an ether bond, a thioether bond, an amide bond, a carbonyl bond and an ester bond) which may have a substituent(s), provided that R² is not the same with R¹ of the formula (1), and an ester bond(s) contained in the formula (2) is/are made 2 or less.

Specific examples and preferred examples of the hydrocarbon group of R² and R³ are the same with the specif examples and preferred examples of the hydrocarbon group of R¹.

The hydrocarbon group of R² and R³ may have a substituent(s), and specific examples and preferred examples of the substituent(s) are the same with the specific examples and preferred examples of the substituent(s) of R¹.

Specific examples and preferred examples of the hydrocarbon group having a substituent(s) of R² and R³ are the same with the specific examples and preferred examples of the hydrocarbon group having a substituent(s) of R¹.

The hydrocarbon group of R² and R³ may be interrupted by a bonding group selected from the group consisting of an ether bond (-O-), a thioether bond (-S-), an amide bond (-CONH-), a carbonyl bond (-C(=O)-) and an ester bond (-C(=O)-O-). Here, the phrase that it may be interrupted by a bonding group selected from the group consisting of the bonding group means that the bonding group(s) exists so as to interrupt the hydrocarbon group. The hydrocarbon group interrupted by the bonding group may further have a substituent(s). The bonding group may be 1 or a plural number. Since the ester bond(s) contained in the formula (2) is/are 2 or less, R² and R³ are each never interrupted by 2 or more ester bonds, and when R² is interrupted by the ester bond, R³ is never interrupted by the ester bond. Incidentally, these groups contain various kinds of isomers.

When it is interrupted by a plural number of the bonding groups, there may be mentioned a hydrocarbon group interrupted by an ester bond, and an ether bond, a thioether bond, an amide bond or a carbonyl bond, and, for example, a linear alkyl group having 2 to 10 carbon atoms or a branched alkyl group having 3 to 10 carbon atoms interrupted by an ester bond, and an ether bond, a thioether bond, an amide bond or a carbonyl bond, and the like.

### Reaction scheme [II]

The ester compound of the formula (2) includes the ester compound of the formula (2a). According to the reaction of the carbonate compound represented by the formula (1) and the ester compound represented by the formula (2a) in the presence of a hydrolase, an asymmetric carbonate compound represented by the formula (3a) and a transesterificated compound represented by the formula (4a) are formed (Reaction scheme [II]). 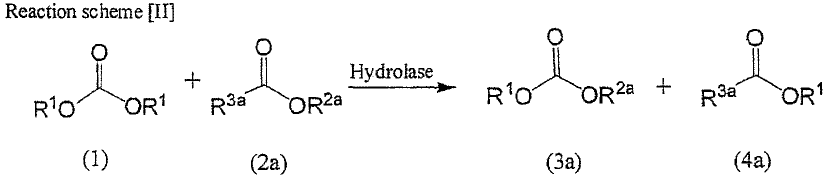 (wherein R¹, R^{2a}, R^{3a} are the same as defined above.)

In Reaction scheme [II], R^{2a} and R^{3a} are each independently, and represent a monovalent hydrocarbon group which may have a substituent(s) (here, a hydrocarbon group of R² and R³ may be interrupted by a bonding group selected from the group consisting of an ether bond, a thioether bond, an amide bond, a carbonyl bond and an ester bond), provided that R^{2a} is not the same as R¹ of the formula (1), and the ester bond(s) contained in the formula (2a) is/are made 2 or less in total.

The hydrocarbon group, substituent(s) and hydrocarbon group having a substituent(s) of R^{2a} are the same as the specific examples and preferred examples of R¹. The hydrocarbon group interrupted by the bonding group of R^{2a} are the same as the specific examples and preferred examples mentioned in R². The hydrocarbon group interrupted by the bonding group may further have a substituent(s).

The hydrocarbon group, substituent(s) and hydrocarbon group having a substituent(s) of R^{3a} are the same as the specific examples and preferred examples of R¹. The hydrocarbon group interrupted by the bonding group of R^{3a} are the same as the specific examples and preferred examples mentioned in R³. The hydrocarbon group interrupted by the bonding group may further have a substituent(s). Here, R^{3a} may be the same or different from R¹ of the formula (I).

### Reaction scheme [III] and [IV]

The formula (2) includes the formula (5a). According to the reaction of the carbonate compound represented by the formula (1) and the ester compound represented by the formula (5a) in the presence of a hydrolase, an asymmetric carbonate compound represented by the formula (6a) is formed (Reaction scheme [III]).

Incidentally, the formed asymmetric carbonate compound represented by the formula (6a) is further reacted with the compound represented by the formula (1) in the presence of a hydrolase, an asymmetric carbonate compound represented by the formula (7a) can be also obtained. The asymmetric carbonate compound represented by the formula (6a) is also included in the formula (2). Also, the formed asymmetric carbonate compound represented by the formula (6a) may be reacted continuously without isolation and purification, etc., with the carbonate compound represented by the formula (1) and the ester compound represented by the formula (5a) whereby the asymmetric carbonate compound represented by the formula (7a) can be directly obtained. 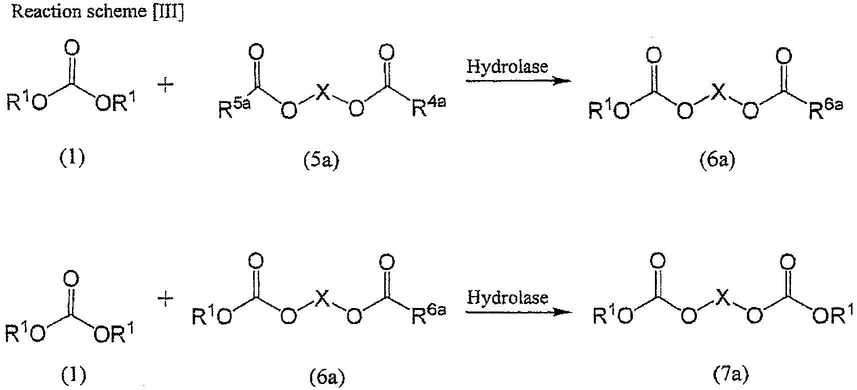 (wherein R¹, R^{4a}, R^{5a}, X and R^{6a} are the same as defined above).

In Reaction scheme [III], R^{4a} and R^{5a} are each independently, and represent a monovalent hydrocarbon group which may have a substituent(s). R^{4a} and R^{5a} are preferably the same.

The hydrocarbon group, substituent(s) and hydrocarbon group having a substituent(s) of R^{4a} are the same as the specific examples and preferred examples mentioned in R¹. Incidentally, R^{4a} may be the same or different from R¹ of the formula (1).

The hydrocarbon group, substituent(s) and hydrocarbon group having a substituent(s) of R^{5a} are the same as the specific examples and preferred examples mentioned in R¹. Incidentally, R^{5a} may be the same or different from R¹ of the **AMENDMENTS FOR PROSECUTION IN THE EPO REGIONAL PHASE** formula (1).

The hydrocarbon group of X may be mentioned, for example, a linear alkylene group having 1 to 20 carbon atoms such as a methylene group, ethylene group, propylene group, butylene group, pentamethylene group, hexamethylene group, etc.; a branched alkylene group having 3 to 20 carbon atoms such as a tetramethylethylene group, etc.; a cycloalkylene group having 3 to 20 carbon atoms such as a cyclopropylene group, cyclobutylene group, cyclopentamethylene group, cyclohexamethylene group, etc.; a hydrocarbon group having 5 to 24 carbon atoms containing 1 or more cycloalkylene group (for example, alhylene-cycloalkylene-alkylene group, etc.); an unsaturated hydrocarbon group having 2 to 20 carbon atoms containing 1 or more ethylene-type unsaturated bond (for example, an alkenylene group having 2 to 20 carbon atoms (for example, vinylene group), an alkadienylene group having 4 to 20 carbon atoms (for example, butadienylene group)); a hydrocarbon group having 7 to 20 carbon atoms containing aromatic skeleton (for example, alkylene-arylene (for example, C₁ to C₁₄ alkylene-phenylene), alkylene-arylene-alkylene (for example, C₁ to C₆ alkylene-phenylene-C₁ to C₆ alkylene (for example, xylylene group)), and the live. Incidentally, these groups contain various kinds of isomers.

X is preferably a linear alkylene group having 1 to 20 carbon atoms; a branched alkylene group having 3 to 6 carbon atoms; a cycloalkylene group having 3 to 6 carbon atoms; an unsaturated hydrocarbon group having 2 to 12 carbon atoms containing 1 or more ethylene-type unsaturated bond (for example, an alkenylene group having 2 to 6 carbon atoms (for example, vinylene group), an alkadienylene group having 4 to 12 carbon atoms (for example, butadienylene group)); a hydrocarbon group having 7 to 12 carbon atoms containing aromatic skeleton (for example, alkylene-arylene (for example, C₁ to C₆ alkylene-phenylene), alkylene-arylene-alkylene (for example, C₁ to C₃ alkylene-phenylene-C₁ to C₃ alkylene (for example, xylylene groups))).

As the substituent(s) for the hydrocarbon group of X, there may be mentioned the same groups of the substituent(s) for the hydrocarbon group mentioned in R¹.

As X having a substituent(s), there may be preferably mentioned a linear alkylene group having 1 to 6 carbon atoms, a branched alkylene group having 3 to 6 carbon atoms, a cycloalkylene group having 3 to 6 carbon atoms or an alkenylene group having 2 to 6 carbon atoms each of which is substituted by a halogen atom(s); an unsaturated hydrocarbon group having 2 to 12 carbon atoms containing 1 or more ethylene-type unsaturated bond and substituted by a halogen atom(s); a hydrocarbon group having 7 to 12 carbon atoms containing aromatic skeleton; a hydrocarbon group having 7 to 12 carbon atoms containing aromatic skeleton and substituted by a halogen atom(s).

The hydrocarbon group of X may be interrupted by a bonding group selected from the group consisting of an ether bond, a thioether bond, an amide bond and a carbonyl bond. The bonding group may be 1 or a plural number.

As X interrupted by the bonding group, there may be preferably mentioned the following:
a hydrocarbon group comprising a linear alkylene group having 2 to 6 carbon atoms or a branched alkylene group having 3 to 6 carbon atoms, or interrupted by an ether bond, phenylene group, naphthylene group or biphenylene group and a linear alkylene group having 1 to 6 carbon atoms or a branched alkylene group having 3 to 6 carbon atoms each of which is interrupted by an ether bond (for example, alkylene-O-arylene (for example, C₁ to C₆ alkylene-O-phenylene));
a linear alkylene group having 2 to 6 carbon atoms or a branched alkylene group having 3 to 6 carbon atoms each of which is interrupted by a thioether bond, or a hydrocarbon group comprising a phenylene group, naphthylene group or biphenylene group and a linear alkylene group having 1 to 6 carbon atoms or a branched alkylene group having 3 to 6 carbon atoms each of which is interrupted by a thioether bond (for example, alkylene-S-arylene (for example, C₁ to C₆ alkylene-S-phenylene));
a linear alkylene group having 2 to 6 carbon atoms or a branched alkylene group having 3 to 6 carbon atoms each of which is interrupted by an amide bond, or a hydrocarbon group comprising a phenylene group, naphthylene group or biphenylene group and a linear alkylene group having 1 to 6 carbon atoms or a branched alkylene group having 3 to 6 carbon atoms each of which is interrupted by an amide bond (for example, alkylene-CONH-arylene, arylene-CONH-C₁ to C₆ alkylene (for example, C₁ to C₆ alkylene-CONH-phenylene, phenylene-CONH-C₁ to C₆ alkylene));
a linear alkylene group having 2 to 6 carbon atoms or a branched alkylene group having 3 to 6 carbon atoms each of which is interrupted by a carbonyl bond, or a hydrocarbon group comprising a phenylene group, naphthylene group or biphenylene group and a linear alkylene group having 1 to 6 carbon atoms or a branched alkylene group having 3 to 6 carbon atoms each of which is interrupted by a carbonyl bond (for example, alkylene-CO-arylene, arylene-CO- alkylene (for example, C₁ to C₆ alkylene-CO-phenylene, phenylene-CO-C₁ to C₆ alkylene)). Incidentally, these groups contain various kinds of isomers.

X is more preferably mentioned a linear alkylene group having 1 to 20 carbon atoms; a branched alkylene group having 3 to 6 carbon atoms; a cycloalkylene group having 3 to 6 carbon atoms; an alkenylene group having 2 to 6 carbon atoms or a linear alkylene group having 1 to 6 carbon atoms or a branched alkylene group having 3 to 6 carbon atoms each of which is interrupted by an ether bond, particularly preferably a linear alkylene group having 1 to 20 carbon atoms; a branched alkylene group having 3 to 6 carbon atoms; an alkenylene group having 2 to 6 carbon atoms or a linear alkylene group having 2 to 6 carbon atoms which is interrupted by an ether bond. Incidentally, these groups contain various kinds of isomers.

### Reaction scheme [IV]

According to the reaction of the carbonate compound represented by the formula (1) and the ester compound represented by the formula (5a) in the presence of a hydrolase, an asymmetric carbonate compound represented by the formula (7a) is formed (Reaction scheme [IV]). 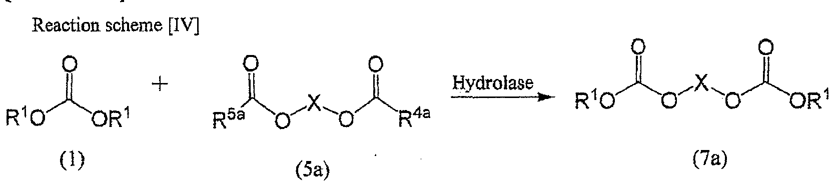 (wherein R¹, R^{4a}, R^{5a} and X are the same as defined above).

The ester compound represented by the formula (5a) is the same as in Reaction scheme [III].

### Reaction scheme [V]

The compound of the formula (2) includes the compound of the formula (8a). According to the reaction of the carbonate compound represented by the formula (1) and the ester compound represented by the formula (8a) in the presence of a hydrolase, an asymmetric carbonate compound represented by the formula (9a) and a transesterificated compound represented by the formula (12a) are formed (Reaction scheme [V]). 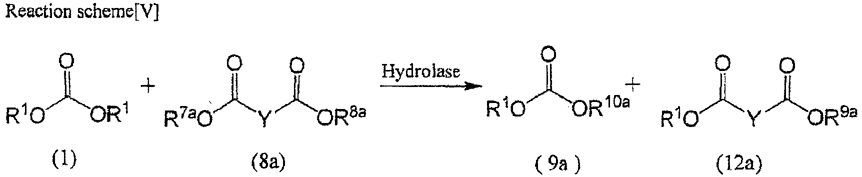 (wherein R^{9a} is R^{7a} or R^{8a}, and R¹, R^{7a}, R^{8a}, Y and R^{10a} are the same as defined above)

In Reaction scheme [V], R^{7a} and R^{8a} are each independently, and represent a monovalent hydrocarbon group which may have a substituent(s). R^{7a} and R^{8a} are preferably the same.

The hydrocarbon group, substituent(s) and hydrocarbon group having a substituent(s) of R^{7a} are the same as the specific examples and preferred examples mentioned in R¹. Incidentally, R^{7a} may be the same or different from R¹ of the formula (1).

The hydrocarbon group, substituent(s) and hydrocarbon group having a substituent(s) of R^{8a} are the same as the specific examples and preferred examples mentioned in R¹. Incidentally, R^{8a} may be the same or different from R¹ of the formula (1).

With regard to Y, the specific examples and preferred examples of X are applied.

### Reaction scheme [VI]

The compound of the formula (2) includes the compound of the formula (10). According to the reaction of the carbonate compound represented by the formula (1) and the cyclic ester compound represented by the formula (10) in the presence of a hydrolase, an asymmetric carbonate compound represented by the formula (11) is formed (Reaction scheme [VI]). 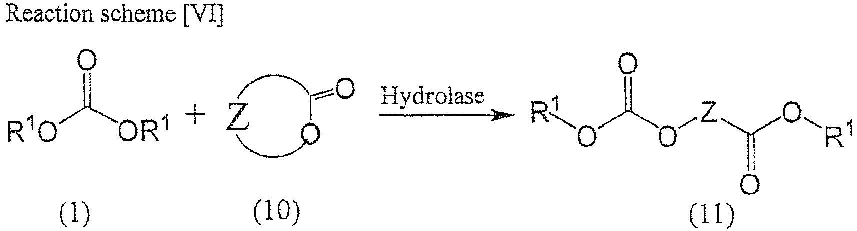 [wherein R¹ and Z are the same as defined above]

In Reaction scheme [VI], the hydrocarbon group of Z may be mentioned, for example, a linear alkylene group having 1 to 12 carbon atoms such as a methylene group, ethylene group, propylene group, butylene group, pentamethylene group, hexamethylene group, etc.; a branched alkylene group having 3 to 12 carbon atoms such as a tetramethylethylene group, etc.; a cycloalkylene group having 3 to 12 carbon atoms such as a cyclopropylene group, cyclobutylene group, cyclopentamethylene group, cyclohexamethylene group, etc.; an alkenylene group haing 2 to 12 carbon atoms such as a vinylene group, etc.; an unsaturated hydrocarbon group having 2 to 12 carbon atoms which gives a compound such as 2-pyrron as the formula (10) (for example, an alkenylene group having 2 to 6 carbon atoms (for example, vinylene group), an alkadienylene group having 4 to 12 carbon atoms (for example, butadienylene group); a hydrocarbon group having 7 to 12 carbon atoms and containing aromatic skeleton which gives a compound such as 3-isochromanone as the formula (10) (for example, alkylene-arylene (for example, C₁ to C₆ alkylene-phenylene), alkylene-arylene-alkylene (for example, C₁ to C₃ alkylene-phenylene-C₁ to C₃ alkylene (for example, xylylene)). Incidentally, these groups contain various kinds of isomers.

The hydrocarbon group of Z may be interrupted by a bonding group selected from the group consisting of an ether bond, a thioether bond, an amide bond, a carbonyl bond and an ester bond. Also, the hydrocarbon group of Z may have a substituent(s). The substituent(s) may be mentioned the same groups as the substituent(s) in R¹.

As the cyclic ester compound represented by the formula (10), there may be mentioned lactones such as p-propiolactone, γ-butyrolactone, δ-valerelactone, γ-valerolactone, ε-caprolactone, α,α-dimethyl-β-propiolactone, β-ethyl-δ-valerolactone, α-methyl-caprolactone, β-methyl-ε-caprolactone, γ-methyl-ε-caprolactone and enanthol-actone, etc.; glycolides such as 1,4-dioxan-2,5-dione and 3,6-dimethyl-1,4-dioxan-2,5-dione, lactides, etc., a cyclic dimer of hydroxycarboxylic acid, etc.

As the cyclic ester compound represented by the formula (10), there may be preferably mentioned δ-valerolactone, ε-caprolactone, γ-butyrolactone, β-propiolactone and 1,4-dioxan-2,5-dione, more preferably δ-valerolactone, β-propiolactone and ε-caprolactone.

An amount of the symmetric carbonate compound represented by the formula (1) to be used in the present invention may be made 0.1 to 100 mol based on 1 mol of the ester compound represented by the formula (2), for example, it can be made 0.5 to 70 mol, above all, it can be made 1 to 50 mol.

In case of Reaction scheme [II], an amount of the symmetric carbonate compound represented by the formula (1) is preferably 0.1 to 100 mol based on 1 mol. of the ester compound represented by the formula (2a), further preferably 0.5 to 50 mol, particularly preferably 1 to 10 mol.

In case of Reaction schemes [III] and [IV], an amount of the symmetric carbonate compound represented by the formula (1) is preferably 0.1 to 100 mol based on 1 mol of the ester compound represented by the formula (5a), further preferably 1 to 70 mol, particularly preferably 5 to 50 mol.

In case of Reaction scheme [V], an amount of the symmetric carbonate compound represented by the formula (1) is preferably 0.1 to 100 mol based on 1 mol of the ester compound represented by the formula (8a), further preferably 0.5 to 50 mol, particularly preferably 1 to 10 mol.

In case of Reaction scheme [VI], an amount of the symmetric carbonate compound represented by the formula (1) is preferably 0.1 to 100 mol based on 1 mol of the cyclic ester compound represented by the formula (7), further preferably 0.3 to 50 mol, particularly preferably 0.5 to 30 mol, most preferably 0.5 to 10 mol.

As the hydrolase to be used in the present invention, there may be mentioned, for example, a protease, esterase, lipase, etc., preferably a pig liver esterase (PLE), a pig pancreas lipase (PPL), a lipase of microorganisms which can be isolated from yeast or bacteria, further preferably a lipase (for example, Amano PS (available from Amano Enzyme Co.). etc.) originated from *Burkholderia cepacia (Pseudomonas cepacia),* a lipase (for example, (Novozym 435 (available from NOVOZYMES)), etc.) originated from *Candida Antarctica*, a lipase (for example, Lipozyme RM IM (available from NOVOZYMES), etc.) originated from *Rhizomucor Wiehei,* a lipase (Lipase TL) originated from *Thermomyces lanuginosus,* a lipase (Lipase MM) originated from *Mucor Miehei,* particularly preferably a lipase originated from *Candida Antarctica* can be used. Incidentally, these hydrolases may be used a commercially available product as a natural form or an immobilized enzyme as such, and may be used alone or in the mixture of two or more.

The hydrolase may be used the commercially available natural form or immobilized enzyme after subjecting to chemical treatment or physical treatment.

As the chemical treatment or physical treatment method, there may be mentioned a method in which, for example, a hydrolase is dissolved in a buffer (an organic solvent may be present, if necessary), and may be used as such, or after stirring, lyophilized, etc. Incidentally, the liophilization herein mentioned means a method in which, for example, an aqueous solution or a substance containing water component is rapidly freezed at a temperature of a freezing point or lower, and a pressure is reduced to a water vapor pressure of the freezed material or lower to remove water by sublime-tion thereof whereby the substance is dried as disclosed in J. Am. Chem. Soc., 122(8), 1565-1571 (2000). According to the treatment, a catalyst activity (reactivity or selectivity, etc.) can be improved.

An amount of the hydrolase to be used in the present invention can be made 1 to 90% by weight based on 1 g of the ester compound represented by the formula (2), for example, it can be made 1 to 70% by weight, above all, it can be made 1 to 50% by weight.

In the case of Reaction scheme [II], an amount of the Hydrolase is preferably 1 to 50% by weight when the amount of the ester compound represented by the formula (2a) is made 100% by weight, further preferably 1 to 30% by weight, particularly preferably 1 to 10% by weight.

In the case of Reaction scheme [III], an amount of the hydrolase is preferably 1 to 50% by weight when the amount of the ester compound represented by the formula (5a) is made 100% by weight, further preferably 1 to 30% by weight, particularly preferably 1 to 10% by weight.

In the case of Reaction scheme [IV], an amount of the hydrolase is preferably 1 to 110% by weight when the amount of the ester compound represented by the formula (5a) is made 100% by weight, further preferably 5 to 70% by weight, particularly preferably 10 to 60% by weight.

In the case of Reaction scheme [V], an amount of the hydrolase is preferably 1 to 50% by weight when the amount of the ester compound represented by the formula (8a) is made 100% by weight, further preferably 1 to 30% by weight, particularly preferably 1 to 10% by weight.

In the case of Reaction scheme [VI], an amount of the hydrolase is preferably 1 to 60% by weight when the amount of the ester compound represented by the formula (7) is made 100% by weight, further preferably 1 to 30% by weight, particularly preferably 1 to 25% by weight.

The reaction of the present invention can be carried out in the absence of a solvent, and may be carried out in an organic solvent. As the organic solvent to be used in the reaction of the present invention, it is not particularly limited so long as it does not deactivate the enzyme, and a dehydrated solvent is desirably used.

As the organic solvent to be used in the reaction of the present invention, there may be mentioned at least one or more selected from, for example, aliphatic hydrocarbons such as n-pentane, n-hexane, n-heptane, n-octane, cyclopentane, cyclohexane and cycloheptane, etc.; aromatic hydrocarbons such as benzene, toluene and xylene, etc.; ethers such as diethyl ether, t-butylmethyl ether, diisopropyl ether, cyclopentylmethyl ether, tetrahydrofuran and 1,4-dioxane, etc.; ketones such as acetone and methyl ethyl ketone, etc.; nitriles such as acetonitrile, propionitrile, etc.

The organic solvent is preferably n-hexane, n-heptane, cyclopentane, cyclohexane, toluene, diisopropyl ether, t-butyl methyl ether, cyclopentylmethyl ether, tetrahydrofuran or acetonitrile, more preferably n-hexane, cyclohexane, toluene, diisopropyl ether, t-butyl methyl ether, cyclopentylmethyl ether or acetonitrile, particularly preferably cyclohexane, toluene or t-butyl methyl ether. Incidentally, these organic solvents may be used alone or in the mixture of two or more kinds.

An amount of the organic solvent to be used is preferably 200 ml or less based on 1 g of the symmetric carbonate compound represented by the formula (1), further preferably 0.1 to 50 ml, particularly preferably 0.3 to 30 ml, most preferably 0.5 to 15 ml.

The reaction of the present invention can be carried out by the method, for example, in the presence or absence of an organic solvent, the symmetric carbonate compound represented by the formula (1), the ester compound represented by the formula (2), and a hydrolase are mixed and reacted under stirring, etc.

A reaction temperature in the reaction of the present invention is preferably 0 to 130°C, further preferably 5 to 100°C, particularly preferably 10 to 80°C. Alto, a reaction pressure is not particularly limited, and preferably carried out under ambient pressure or under reduced pressure.

The reaction of the present invention is a so-called reversible reaction. In case of Reaction scheme [II], there is a case where the by-producing transesterificated compound represented by the formula (4a) has a lower boiling point than those of the symmetric carbonate compound represented by the formula (1) and the ester compound represented by the formula (2a) as starting materials, and the asymmetric carbonate compound represented by the formula (3a) as a reaction product. Thus, by carrying out the reaction while removing the by-producing transesterificated compound represented by the formula (4a) outside the reaction system, the reaction rapidly proceeds by moving the chemical equilibrium to the product side, whereby the target asymmetric carbonate compound represented by the formula (3a) can be obtained more effectively, so that this is mentioned as one of the preferred embodiments of the present invention.

This method can be accomplished by, for example, optionally selecting the substituents R³, R^{2a} and R^{3a} so as to by-produce the transesterificated compound represented by the formula (4a) having a lower boiling point than those of the symmetric carbonate compound represented by the formula (1) and the ester compound represented by the formula (2a) as starting materials, and that of the asymmetric carbonate compound represented by the formula (3a) as a product.

More specifically, in Reaction scheme [II], when R¹ and R^{3a} are both methyl groups, and R^{2a} is other than a methyl group, the transesterificated compound represented by the formula (4a) (methyl acetate, boiling point; 57-58°C) formed by the reaction is expected to have a lower boiling point than those of the starting symmetric carbonate compound represented by the formula (1) and the ester compound represented by the formula (2a). Thus, at the time of the reaction, by optionally regulating a reaction temperature, and/or a reduced degree of pressure (0.13 kPa or higher, and less than 101.3 kPa (1.0 mmHg or higher, and less than 760 mmHg), preferably pressure reducing degree (exceeding 1.3 kPa, and less than 101.3 kPa (exceeding 10 mmHg, and less than 760 mmHg), it is possible to produce the target compound while selectively removing the transesterificated compound represented by the formula (4) to outside of the reaction system. As a result, the equilibrium of the reaction moves to the product side and the present reaction can proceed rapidly, whereby the asymmetric carbonate compound represented by the formula (3a) which is the target product can be obtained within a shorter reaction time.

The production apparatus(s) is/are not particularly limited, and, for example, the reaction can be carried out by a conventional production apparatus(s) such as a reaction vessel, heating (cooling) apparatus, etc. Moreover, when the transesterificated compound represented by the formula (4a) is to be removed from the reaction vessel, there may be preferably mentioned, for examples, a production apparatus equipped with a distillation device having a rectification device, etc., and a recovery device of the distillate, etc., more preferably, in addition to the above-mentioned distillation device and recovery device, etc., a production apparatus equipped with a device which can adjust a reaction pressure.

Also, among the distillates recovered by the recovery device, the transesterificated compound represented by the formula (4a) and unreacted starting compounds may be used again for the reaction of the present invention.

Moreover, the obtained asymmetric carbonate compound may be further purified by the conventional methods such as distillation, liquid separation, extraction, crystallization, recrystallization and column chromatography, etc.

The asymmetric carbonate compound obtained by the preparation process of the present invention by using a hydrolase, is a chemically safer product, because of the product is extremely less contaminated by impurities, such as a metal salt or a halide ,etc. which could occur in the conventional preparation method.

### EXAMPLES

Next, the present invention is explained more specifically by referring to Examples, but the scope of the present invention is not limited by these.

With regard to the obtained target compound, measurement of purity was carried out by using IR, NMR spectr-um analysis, etc., and other gas chromatography. Incidentally, the analytical conditions of the gas chromatography are as follows.

Analytical conditions of gas chromatography;
Analytical device: GC-17A (manufactured by SHIMADZU)
Column used: DB-WAX (manufactured by J&W); 0.53 mm LD. *30m, Layer thickness 1 qm
Analytical temperature: Starting temperature: 40°C (maintained for 10 minutes),
   Finishing temperature 230°C (maintained for 31 minutes)
Temperature raising rate: 10°C/min
Carrier gas: helium, 11.13 ml /min
Injection port: No split, 230°C
Detector: FID, 230°C

### Example 1 (Reaction scheme [II]: Synthesis of n-butylmethyl carbonate)

In an apparatus made of glass having an inner volume of 100 ml and equipped with a stirring device, a thermometer and a condenser were charged 20.0 g (172 mol) of n-butyl acetate, 72.5 ml (860 mol) of dimethyl carbonate, and 1.00 g of lipase derived from *Candida Antarctica* (Novozym 435 (Trade Name); available from NOVOZYMES) and mixed, and the mixture was reacted under stirring at 50°C for 63 hours. After completion of the reaction, the reaction solution was filtered, and the filtrate was washed with 50 ml of dimethyl carbonate. After condensing the filtrate, the condensate was purified by distillation under reduced pressure, to obtain 7.45 g of n-butylmethyl carbonate (purity (Areal percentage by gas chromatography); 96.8%, isolation yield based on n-butyl acetate; 33%) as colorless liquid.

Physical properties of the obtained n-butylmethyl carbonate were as follows. CI-MS (m/z); 13 3[M+1].
¹H-NMR (CDCl₃, δ (ppm)); 0.94 (3H, t, J=7.32Hz), 1.40 (2H, tq, J=7.32Hz), 1.66 (2H, tt, J=6.59Hz), 3.78 (3H, s), 4.14 (2H, t, J=6.59Hz).
'3C-NMR (CDCl3, 6 (ppm)); 13.7, 189, 30.7, 54.6, 68.0, 155.9.
EA; Calcd: C, 54.53%; H, 9.15%; O, 36.32%
Found: C, 53.69%; H, 8.96%.

### Example 2 (Reaction scheme [II]: Synthesis of n-butylmethyl carbonate)

In an apparatus made of glass having an inner volume of 20 ml and equipped with a stirring device and a thermometer were charged 1.00 g (8.61 mmol) of n-butyl acetate, 3.63 ml (43.1 mmol) of dimethyl carbonate, and 50.7 mg af a lipase derived from *Candida Antarctica* (Novozym 435 (Trade Name); available from NOVOZYMES) and mixed, and the mixture was reacted under stirring at 30°C for 33 hours. After completion of the reaction, the obtained reaction solution was filtered, and the filtrate was washed with 6 Il of dimethylsulfoxide. When the obtained filtrate was analyzed by gas chromatography (Internal standard method), then, 0.836 g of n-butylmethyl carbonate was found to be formed (Reaction yield based on n-butyl acetate; 73%).

### Example 3 (Reaction scheme [II]: Synthesis of n-butylmethyl carbonate)

In the same manner as in Example 2 except for changing an amount of dimethyl carbonate used is made 0.784 g (8.70 mol), and adding 3 ml of cyclohexane, the same procedure was carried out and reaction was carried out at 30°C for 24 hours. After completion of the reaction, the obtained reaction solution was filtered, and the filtrate was obtained. When the obtained filtrate was analyzed by gas chromatography (Internal standard method), then, 0.394 g ofn-butylmethyl carbonate was found to be formed (Reaction yield based on n-butyl acetate; 35%).

### Example 4 (Reaction scheme [II]: Synthesis of n-butylmethyl carbonate)

In the same manner as in Example 3 except for using t-butyl methyl ether in place of cyclohexane, the same procedure was carried out and reaction was carried out at 30°C for 24 hours. After completion of the reaction, the obtained reaction solution was filtered. When the obtained filtrate was analyzed by gas chromatography (Internal standard method), 0.378 g af n-butylmethyl carbonate was found to be formed (Reaction yield; 33%).

### Example 5 (Reaction scheme [II]: Synthesis of ethylmethyl carbonate)

In an apparatus made of glass having an inner volume of 20 ml and equipped with a stirring device and a thermometer were charged 0.504 g (5.72 mmol) of ethyl acetate, 4.78 ml (56.7 mmol) of dimethyl carbonate, and 25.0 mg of a lipase derived from *Candida Antartica* ((Novozym 435 (Trade Name); available from NOVOZYMES) and mixed, and the mixture was reacted under stirring at 30°C for 80 hours. After completion of the reaction, when the obtained reaction mixture was analyzed by gas chromatography (Internal standard method), then, 0.417 g of ethylmethyl carbonate was found to be formed (Reaction yield based on ethyl acetate; 70%).

### Example 6 (Reaction scheme [II]: Synthesis of cyclohexylmethyl carbonate)

In an apparatus made of glass having an inner volume of 20 ml and equipped with a stirring device and a thermometer were charged 1.01 g (7.10 mol) of cyclohexyl acetate, 2.96 ml (35.1 mmol) of dimethyl carbonate, and 50.2 mg of a lipase derived from *Candida Antarctica* (Novozynn 435 (tirade Nave); available from NOVOZYMES) and mixed, and the mixture was reacted under stirring at 30°C for 98 hours, further by adding 49.7 mg of the same lipase at the same temperature for 98 hours, thereafter, furter adding 49.9 mg of the same to the mixture and the mixture was reacted for 160 hours. After completion of the reaction, the obtained reaction solution was filtered, and the filtrate was washed with 40 ml of t-butyl methyl ether. The obtained filtrate was washed twice with 5 ml of saturated aqueous solution of NaCI, the organic layer was extracted and dried by using anhydrous magnesium sulfate. When the obtained organic layer was condensed, 0.686 g of cyclohexylmethyl carbonate (purity (Areal percentage by gas chromatography); 85.6%, Isolation yield based on cyclohexyl acetate; 52%) was obtained as colorless liquid.

Physical properties of the obtained cyclohexylmethyl carbonate are as follows. CI-MS (m/z); 159 [M+1].
¹H-NMR (CDCl3, δ (ppm)); 1.18-1.59 (6H. m), 1.70-1.80 (2H, m), 1.87-1.97 (2H, m), 3.77 (3H, s), 4.62 (1H, tt, J=3.91, 9.16Hz).
¹³C-NMR (CDCl₃, δ (ppm)); 23.6, 25.3, 31.6, 54.4, 76.8, 155.3.
IR (Nujol, cm⁴); 793, 951, 1013, 1035, 1259, 1277, 1320, 1444, 1746, 2861, 2940.

### Example 7 (Reaction scheme [II]: Synthesis of ethylmethyl carbonate)

In an apparatus made of glass for consinuous synthetic apparatus were charged 6.47 g (54.7 mmol) of diethyl carbonate, 1.01 g (13.6 mmol) of methyl acetate, and 50.6 mg of a lipase derived from *Candida Antarctica* (Novozym 435 (Trade Name); available from NOVOZYMES) and mixed, and the mixture was reacted under stirring at 40°C for 22 hours. After completion of the reaction, the obtained reaction solution was filtered, and the filtrate was obtained. When the obtained filtrate was analyzed by gas chromatography (Internal standard method), then, 0.57 g of ethylmethyl carbonate was found to be formed (Reaction yield; 40.2%).

### Comparative example 1 (Reaction scheme [II]: Synthesis af n-butylmetlayl carbonate; no enzyme)

In an apparatus made of glass having an inner volume of 20 ml and equipped with a stirring device, a condenser and a thermometer were charged 1.00 g (8.61 mmol) of n-butyl acetate and 3.63 ml (43.1 mol) of dimethyl carbonate and mixed, and the mixture was reacted under stirring at 100°C for 24 hours. After completion of the reaction, the obtained reaction solution was filtered, and when the obtained filtrate was analyzed by gas chromatography, no n-butylmethyl carbonate was found to be formed.

### Example 8 (Reaction scheme [V]: Synthesis of ethylmethyl carbonate)

In an apparatus made of glass having an inner volume of 10 ml and equipped with a stirring device, a thermometer and a condenser were charged 0.70 g (4.0 mmol) of dimethyl adipate, 4.75 g (40.0 mmol) of diethyl carbonate, and 0.035 g of a lipase derived from *Candida Antarctica* (Novozym 435(Trade Name); available from NOVOZYMES) and mixed, and the mixture was reacted under stirring at 40°C for 24 hours. After completion of the reaction, the obtained reaction solution was filtered, and the filtrate was obtained. When the obtained filtrate was analyzed by gas chromatography (Internal standard method), then, 0.47 g of ethylmethyl carbonate was found to be formed (Reaction yield based on dimethyl adipate; 56.2%).

### Example 9 (Reaction scheme [IV]: Synthesis of 1,2-dimethoxycarbonyloxyethane)

In an apparatus made of glass having an inner volume of 50 ml and equipped with a stirring device, a thermometer and a condenser were charged 1.00 g (6.84 mmol) of ethyleneglycol diacetate, 28.8 ml (342 mmol) of dimethyl carbonate, and 500 mg of a lipase derived from *Candida Antarctica* (Novozym 435 (Trade Name); available from NOVOZYMES) and mixed, and the mixture was reacted under stirring at room temperature for 15 hours. After completion of the reaction, the reaction solution was filtered, and the filtrate was further washed with 5 ml of dimethyl carbonate to obtain a filtrate. When the obtained filtrate was condensed, 1.10 g of 1,2-dimethoxycarbonyl-oxyethane (purity (Areal percentage by gas chromatography); 84.4%, Isolation yield based on ethyleneglycol diacetate; 76%) was obtained as colorless liquid.

Physical properties of the obtained 1,2-dimethoxycarbonyloxyethane are as follows.
CI-MS (m/z); 179 [M+1].
¹H-NMR (CDCl₃, 1320, (ppm)); 3.80 (6H, s), 4.37 (4H, s).
¹³C-NMR (CDCl₃, δ (ppm)); 55.0, 18.65.4.155.6.
IR (Nujol, cm⁻¹); 791, 866, 947, 1032, 1254, 1299, 1346, 1376, 1405, 1445, 1754, 2963, 3008.
EA; Calcd: C, 40.45%; H, 5.66%
Found: C, 40.64%; H, 5.40%.

### Example 10 (Reaction scheme [IV]: Synthesis of 1,2-diethoxycarbonyloxyethane)

In an apparatus made of glass having an inner volume of 50 ml and equipped with a stirring device, a thermometer and a condenser were charged 400 mg (2.74 mol) of ethyleneglycol diacetate, 16.6 ml (137 mmol) of diethyl carbonate, and 200 mg of a lipase derived from *Candida Antarctica* (Novozym 435(Trade Name); available from NOVOZYMES) and mixed, and the mixture was reacted under stirring at room temperature for 15 hours. Further, 200 mg of a lipase derived from *Candida Antarctica* (Novozym 435(Trade Name); available from NOVOZYMES) was mixed with the reaction mixture, the mixture was reacted under stirring at room temperature for 24 hours.

After completion of the reaction, the reaction solution was filtered, and the filtrate was washed with 5 ml of acetonitrile to obtain a filtrate. When the obtained filtrate was condensed, then, 522 mg of 1,2-diethoxycarbonyloxyethane (purity (Areal percentage by gas chromatography); 86.5%, Isolation yield based on ethyleneglycol diacetate; 80%) was obtained as colorless liquid.

Physical properties of the obtained 1,2-diethoxycarbonyloxyethane are as follows.
CI-MS (m/z); 207 [M+1].
¹H-NMR (CDCl3, δ (ppm)); 1.31 (4H, t, J=7.08Hz), 4.21 (6H, q, J=7.08Hz), 4.36 (4H, s).
¹³C-NMR (CDCl₃, δ (ppm)); 64.3, 65.2, 154.9.
IR (Nujol, cm⁻¹); 474, 790, 858, 882, 1008, 1033, 1092, 1115, 1177, 1243, 1287, 1341, 1380, 1402, 1448, 1583, 1749. 2914, 2987.
EA; Calcd: C, 46.60%; H, 6.84%
Found: C, 46.93%; H, 6.70%.

### Example 11 (Reaction scheme [IV]: Synthesis of 1,3-dimethoxycarbonyloxypropane)

In an apparatus made of glass having an inner volume of 50 ml and equipped with a stirring device, a thermometer and a condenser were charged 1.00 g (6.24 mol) of 1,3-propanedioldiacetate, 26.0 ml (309 mmol) of dimethyl carbonate, and 500 mg of a lipase derived from *Candida Antarctica* (Novozym 435(Trade Name); available from NOVOZYMES) and mixed, and the mixture was reacted under stirring at room temperature for 48 hours. After completion of the reaction, the reaction solution was filtered, and the filtrate was washed with 5 ml of dimethyl carbonate to obtain a filtrate. When the obtained filtrate was condensed, then, 1.13 g of 1,3-dimethoxycarbonyl-oxypropane (purity (Areal percentage by gas chromatography); 87.7%, Isolation yield based on 1,3-propanedioldiacetate; 83%) was obtained as colorless liquid.

Physical properties of the obtained 1,3-dimethoxycarbonyloxypropane are as follows.
CI-MS (m/z), 193 [M+1].
¹H-NMR (CDCl₃. δ (ppm)); 2.05 (2H, tt, J=6.35, 6.35Hz), 3.79 (6H, s), 4.29 (4H, t, J=6.35Hz).
¹³C-NMR (CDCl3, δ (ppm)); 28.2, 54.8, 64.3, 155.7.
IR (Nujol, cm⁻¹); 457, 793, 907, 941, 976, 1028, 1114, 1259, 1332, 1363, 1389, 1445, 1750,2911,2963.
EA; Calcd: C, 43.75%; H, 6.29%
Found: C, 44.20%; H, 6.17%.

### Example 12 (Reaction scheme [IV]: Synthesis of 1,4-dimethoxycarbonyloxybutane)

In an apparatus made of glass having an inner volume of 50 ml and equipped with a stirring device, a thermometer and a condenser were charged 1.00 g (5.74 mmol) of 1,4-butanedioldiacetate, 24.0 ml (285 mmol) of dimethyl carbonate, and 500 mg of a lipase derived from *Candida Antar ctica* (Novozym 435(Trade Name); available from NOVOZYMES) and mixed, and the mixture was reacted under stirring at room temperature for 48 hours. After completion of the reaction, the reaction solution was filtered, and the filtrate was washed with 5 ml of acetonitrile to obtain a filtrate. When the obtained filtrate was condensed, then, 1.12 g of 1,4-dimethoxycarbonyloxybutane (purity (Areal percentage by gas chromatography); 94.3%, Isolation yield based on 1,4-butanedioldiacetate; 89%) was obtained as white solid.

Physical properties of the obtained 1,4-dimethoxycarbonyloxybutane are as follows.
CI-MS (m/z); 207 [M+1].
¹H-NMR (CDCl3, δ (ppm)); 1.78 (4H, m), 3.78 (6H, s), 4.18 (4H, m).
¹³C-NMR (CDCl3, δ (ppm)); 25.2, 54.7, 67.4, 155.8.
IR (KBr method, cm⁻¹); 558, 721, 743, 795, 934, 959, 1055, 1111, 1249, 1292, 1405. 1446, 1484, 1753, 2864, 2886, 2910, 2983, 3022, 3467.
EA; Calcd: C, 46.60%; H, 6.84%
Found: C, 47.13%; H, 6.70%.

### Example 13 (Reaction scheme [IV]: Synthesis of 1,6-dimethoxycarbonyloxyhexane)

In an apparatus made of glass having an inner volume of 50 ml and equipped with a stirring device, a thermometer and a condenser were charged 1.00 g (4.94 mmol) of 1,6-hexanedioldiacetate, 21.0 ml (249 mmol) of dimethyl carbonate, and 500 mg of a lipase derived from *Candida Antarctica* ((Novozym 435 (Trade Name); available from NOVOZYMES) and mixed, and the mixture was reacted under stirring at room temperature for 48 hours. After completion of the reaction, the reaction solution was filtered, and the filtrate was washed with 5 ml of acetonitrile to obtain a filtrate. When the obtained filtrate was condensed, then, 1.09 g of 1,6-dimethoxycarbonyloxyhexane (purity (Areal percentage by gas chromatography); 91.1%, Isolation yield based on 1,6-hexanediol diacetate; 86%) was obtained as white solid.

Physical properties of the obtained 1,6-dimethoxycarbonyloxyhexane are as follows.
CI-MS (m/z); 235 [M+1].
¹H-NMR (CDCl₃, δ (ppm)); 1.42 (4H, m), 1.68 (4H, m), 3.77 (6H, s), 4.14 (4H, t, J=6,59Hz).
¹³C-NMR (CDCl₃, δ (ppm)); 25.4, 28.6, 54.6, 68.0, 155.9.
IR (KBr method, cm⁻¹); 422, 572, 721, 791, 803, 932, 957, 1027, 1076, 1112, 1291, 1348,1403,11445,1485, 1702, 1755,2875,2924,2943,2969, 3020,3467.
EA; Calcd: C, 51.27%; H, 7.75%
Found: C, 51.23%; H, 7.56%.

### Example 14 (Reaction scheme [IV]: Synthesis of 1,4-dimethoxycarbonyloxy-cis-2-butene)

In an apparatus made of glass having an inner volume of 200 ml and equipped with a stirring device were charged 5.14 g (30 mmol) of cis-2-butene-1,4-dioldiacetate, 26.00 g (288 mmol) of dimethyl carbonate, and 0.5 g of a lipase derived from *Candida Antartica* (Novozym 435(Trade Name); available from NOVOZYMES) and mixed, and the mixture was reacted under stirring at 70°C for 69 hours, further stirred by adding 2 g of dimethyl carbonate at 70°C for 7 hours, and then, reacted under the conditions of reduced pressure (750 hPa), at 70°C for 5 hours. After completion of the reaction, the reaction mixture was filtered while washing with dimethyl carbonate to obtain a filtrate. The obtained filtrate was condensed, a part (3.08 g) was rectified by kugelrohr distillation, and when an amount of the product was confirmed, then, 2.54 g of 1,4-dimethoxycarbonyloxy-cis-2-butene (Yield based on cis-2-butene-1,4-diol diacetate; 42%) was found to be contained in the whole filtrate.

Physical properties of the obtained 1,4-dimethoxycarbonyloxy-cis-2-butene are as follows.
CI-MS (m/z); 129 [M-OC(O)OMe]
¹H-NMR (CDCl₃, δ (ppm)); 5.81 (2H, m, J=1.22Hz), 4.74-4.76 (4H, dd, J=1.46, 4.15Hz), 3.79 (6H, s).

### Example 15 (Reaction scheme [IV]: Synthesis of 1-methoxycarbonyloxy-2-(methoxy-carbonyloxyethoxy)ethane)

In an apparatus made of glass having an inner volume of 200 ml and equipped with a stirring device were charged 25.27 g (132 mmol) of diethyleneglycol diacetate, 59.2 g (657 mmol) of dimethyl carbonate, and 7.56 g of a lipase derived from *Candida Antartica* (Novozym 435(Trade Name); available from NOVOZYMES) and mixed, and the mixture was reacted under stirring at 70°C for 68 hours. The reaction mixture was condensed in the presence of the enzyme, and 38.85 g of dimethyl carbonate was additionally added thereto and the mixture was stirred at 70°C for 24 hours. After stirring, the reaction mixture was condensed in the presence of the enzyme, 30.1 g of dimethyl carbonate was additionally added, and the mixture was stirred at 70°C for 24 hours. After completion of the reaction, the reaction mixture was filtered while washing with dimethyl carbonate to obtain a filtrate. The obtained filtrate was condensed, and a part (3.79 g) thereof was purified by kugelrohr distillation. When an amount of the product was confirmed, then, 14.66 g of 1-methoxycarbonyloxy-2-(methoxycarbonyloxyethoxy)ethane (Isolation yield based on diethyleneglycol diacetate; 53%) was found to be contained in the whole filtrate.

Physical properties of the obtained 1-methoxycarbonyloxy-2-(methoxy-carbonyloxyethoxy)ethane are as follows.
CI-MS (m/z); 223 [M+1]
¹H-NMR (CDCl₃, δ (ppm)); 4.27-4.30 (4H, t, J=4.63Hz), 3.79 (6H, s), 3.71-3.74 (4H, t, J=4.63Hz).

### Example 16 (Reaction scheme [IV]: Synthesis of 1,12-dimethoxycarbonyloxydodecane)

In an apparatus made of glass having an inner volume of 200 ml and equipped with a stirring device were charged 20.42 g (71 mol) of 1,12-dodecanedioldiacetate, 31.35 g (348 mmol) of dimethyl carbonate, and 3.77 g of a lipase derived from *Candida Antarctica* (Novozym 435(Trade Name); available from NOVOZYMES) and mixed, and the mixture was reacted under stirring at 70°C for 48 hours. The reaction mixture was condensed in the presence of the enzyme, 30.24 g of dimethyl carbonate was additionally added to the condensate and the mixture was stirred at 70°C for 24 hours. After stirring, the reaction mixture was condensed in the presence of the enzyme, 30.22 g of dimethyl carbonate was additionally added to the condensate and the mixture was stirred at 70°C for 25 hours. The reaction mixture was condensed in the presence of the enzyme, 30.23 g of dimethyl carbonate was additionally added to the condensate and the mixture was stirred at 70°C for 20 hours. After completion of the reaction, the reaction mixture was filtered while washing with dimethyl carbonate and tetrahydrofuran to obtain a filtrate. The obtained filtrate was condensed, and when a part of condensate (1.17 g) was purifed by kugelrohr distillation and an amount of the formed product was confirmed, finally, 14.38 g of 1,12-dimethoxycarbonyloxydodecane (Yield based on 1,12-dodecanedioldiacetate; 63%) was found to be contained in the whole filtrate.

Physical properties of the obtained 1,12-dimethoxycarbonyloxydodecane are as follows.
CI-MS (m/z), 319 [M+1]
¹H-NMR (CDCl₃, δ (ppm)); 4.10-4.15 (4H, t, J=6.83Hz), 3.77 (6H, s), 1.61-1.70 (4H,
m, J=6.59Hz), 1.26-1.38 (16H, m).

### Example 17 (Reaction scheme (VI]: Synthesis of methyl 6-methoxycarbonyloxyhexanoate)

In an apparatus made of glass having an inner volume of 20 ml and equipped with a stirring device and a thermometer were charged 1.01 g (8.85 mol) of s-caprolactone, 3.94 g (43.7 mmol) of dimethyl carbonate and 51.1mg of a lipase derived from *Candida Antarctica* (Novozym 435(Trade Name); available from NOVOZYMES) and mixed, and the mixture was reacted under stirring at 30°C for 186 hours. After completion of the reaction, the obtained reaction solution was filtered, and the filtrate was washed with 30 ml of dimethyl carbonate to obtain a filtrate. When the obtained filtrate was condensed, finally, 1.68 g of methyl 6-methoxycarbonyloxyhexanoate (purity Areal percentage by gas chromatography); 84.0%, Isolation yield based on ε-caprolactone; 78%) was obtained as colorless liquid.

Physical properties of the obtained methyl 6-methoxycarbonyloxyhexanoate are as follows.
CI-MS (m/z); 205 [M+1].
¹H-NMR (CDCl₃, δ (ppm)); 1.35 to 1.47 (2H, m), 1.61 to 1.74 (4H, m), 2.33 (2H, t, J=7.45Hz), 3.67 (3H, s), 3.78 (3H, s), 4.14 (2H, t, J=6.59Hz).
¹³C-NMR (CDCl₃, δ (ppm)); 24.5,25.3, 28.4, 33.9, 51.5, 54.7, 67.9, 155.9,173.9

IR (Nujol, cm⁻¹); 794, 957, 1013, 1103, 1168, 1200, 1269, 1363, 1388. 1443, 1747, 2866, 2956.

### Example 18 (Reaction scheme [VI]: Synthesis of methyl 6-methoxycarbonyloxyhexanoate)

In an apparatus made of glass having an inner volume of 500 ml and equipped with a stirring device, a thermometer and a reflux condenser were charged 22.8 g (0.200mol) of ε-capralactone, 90.1 g (1.00mol) of dimethyl carbonate and 2.28 g of a lipase derived from *Candida Antartica* (Novozym 435(Trade Name); available from NOVOZYMES) and mixed, and the mixture was reacted under stirring at 60°C for 31 hours. 45.1 g (0.501mol,) of dimethyl carbonate was further added to the mixture, and the mixture was reacted at the same temperature for 7 hours. After completion of the reaction, the obtained reaction mixture was cooled to room temperature, and then filtered. When the obtained filtrate was purified by distillation under reduced pressure, finally, 17.8 g of methyl 6-methoxycarbonyloxyhexanoate (purity (Areal percentage by gas chromatography); 98.0%, Isolation yield based on ε-caprolactone; 43%) was obtained as colorless liquid (138 to 139°C/1.6 to 1.7 kPa).

### Example 19 (Reaction scheme [VI]: Synthesis of methyl 6-methoxycarbonyloxyhexanoate)

In an apparatus made of glass having an inner volume of 500 ml and equipped with a stirring device, a thermometer and a reflux condenser were charged 45.6 g (0.400mol) of ε-caprolactone, 180 g (2.00mol) of dimethyl carbonate and 9.12 g of a lipase derived from *Candida Antarctica* (Novozym 435(Trade Name); available from NOVOZYMES) and mixed, and the mixture was reacted under stirring at 80°C for 23 hours. After completion of the reaction, the obtained reaction mixture was cooled to room temperature, and 90.1 g (1.00mol) dimethyl carbonate was added to the mixture and the mixture was filtered. When the obtained filtrate was analyzed by gas chromatography (Internal standard method), finally, 66.9 g of methyl 6-methoxy-carbonyloxyhexanoate (Reaction yield based on ε-caprolactone; 82%) was found to be formed.

### Example 20 (Reaction scheme [VI]: Synthesis of methyl 6-methoxycarbonyloxyhexanoate)

In the same manner as in Example 19 except for changing an amount of dimethyl carbonate to be used to 360 g (4.00 mol), the same procedure was carried out and reacted at 80°C for 48 hours. After completion of the reaction, the obtained reaction mixture was cooled to room temperature, and then filtered. When the obtained filtrate was quantitatively analyzed by gas chromatography (Internal standard method), finally, 71.3 g of methyl 6-methoxycarbonyloxyhexanoate (Reaction yield based on ε-caprolactone; 87%) was found to be formed.

### Example 21 (Reaction scheme [VI]: methyl 5-methoxycarbonyloxypentanoate)

In an apparatus made of glass having an inner volume of 200 ml and equipped with a stirring device, a thermometer and a reflux condenser were charged 25.0 g (0.25mol) of δ-valerolactone, 112.5 g (1.25mol) of dimethyl carbonate and 5.00 g of a lipase derived from *Candida Antarctica* (Novozym 435(Trade Name); available from NOVOZYMES) and mixed, and the mixture was reacted under stirring at 60°C for 35 hours. After completion of the reaction, the obtained reaction mixture was cooled to room temperature, and then filtered. The obtained filtrate was condensed under reduced pressure to obtain 52.3 g of a condensate. When the condensate was quantitatively analyzed by Internal standard method using gas chromatography, then, 36.6 g of methyl 5-methoxycarbonyloxypentanoate (Reaction yield based on δ-valerolactone; 77.0%) was found to be formed. When the condensate was purified by distillation under reduced pressure, finally, 27.0 g of methyl 5-methoxycarbonyloxypentanoate (purity (Areal percentage by gas chromatography); 98.8%, Isolation yield based on δ-valerolactone; 56.1%) was obtained as colorless liquid (106 to 107°C/0.9 to 1.1 kPa).

Physical properties of the obtained methyl 5-methoxycarbonyloxypentanoate are as follows.
CI-MS (m/z); 191 [M+1]
¹H-NMR (CDCl₃, δ (ppm)); 1.70 to 1.75 (4H, m), 2.34 to 2.39 (2H, m), 3.67 (3H, s), 3.78 (3H, s), 4.13 to 4.18 (2H, m).

### Example 22 (Reaction scheme [VI]: methyl 3-methoxycarbonyloxypropionate)

In an apparatus made of glass having an inner volume of 300 ml and equipped with a stirring device, a thermometer and a reflux condenser were charged 28.6 g (0.38mol) of β-propiolactone with a purity of 95%, 178.7 g (1.98mol) of dimethyl carbonate and 5.72 g of a lipase derived from *Candida Antarctica* (Novozym 435(Trade Name); available from NOVOZYMES) and mixed, and the mixture was reacted under stirring at 60°C for 14.5 hours. Thereafter, 5.72 g of a lipase derived from *Candida Antartica* (Novozym 435(Trade Name); available from NOVOZYMES) was further added to the mixture, and the mixture was reacted at 60°C for 40 hours. After completion of the reaction, the obtained reaction mixture was cooled to room temperature, and then filtered. The obtained filtrate was condensed under reduced pressure, to obtain 70.5 g of a condensate. When the condensate was determined (internal standard method) by gas chromatography, 42.8 g of methyl 3-methoxycarbonyloxypropionate was found to be formed (Reaction yield based on β-propiolactone; 69.5%).

Finally, when the condensate was purified by distillation under reduced pressure, 1320, g of methyl 3-methoxycarbonyloxypropionate (purity (Areal percentage by gas chromatography); 97.3%, Isolation yield based on β-propiolactone; 51.5%) was obtained as colorless liquid (81 to 83°C/0.7 to 0.8 kPa).

Physical properties of the obtained methyl 3-methoxycarbonyloxypropionate are as follows.
CI-MS (m/z); 163 [M+1]
¹H-NMR (CDCl₃, δ (ppm)); 2.68 to 2.73 (2H, t, J=6.35Hz), 3.71 (3H, s), 3.79 (3H, s), 4.36 to 4.44 (2H, t, J=6.35Hz).

### Example 23 (Reaction scheme [III]: 4-methoxycarbonyloxybutylacetate)

In an apparatus made of glass having an inner volume of 200 ml and equipped with a stirring device, a thermometer and a reflux condenser were charged 174.2 g (1.00mol) of 1,4-butanedioldiacetate, 180.0 g (2.00mol) of dimethyl carbonate and 8.70 g of a lipase derived from *Candida Antarctica* (Novozym 435(Trade Name); available from NOVOZYMES) and mixed, and the mixture was reacted under stirring at 70°C for 32 hours. After completion of the reaction, the obtained reaction mixture was cooled to room temperature, and then filtered. The obtained filtrate was condensed under reduced pressure to obtain 196.0 g of a condensate. When the condensate was purified by distillation under reduced pressure, finally, 10.6 g of 4-methoxycarbonyloxybutyl-acetate (purity: (Areal percentage by gas chromatography); 79.2%) was obtained as colorless liquid (99 to 103°C/0.6 to 0.8 kPa).

Physical properties of the obtained 4-methoxycarbonyloxybutylacetate are as follows.
CI-MS (m/z); 191[M+ 1]
¹H-NMR (CDCl_{3,}δ(ppm)); 1.71 to 1.79(4H,m), 2.05(3H,s), 3.78(3H,s), 4.07 to 4.12(2H,t,J=6.23Hz), 4.15 to 4.20(2H,t,J=6.23Hz).

### UTILIZABILITY IN INDUSTRY

The present invention relates to a process for obtaining an asymmetric carbonate compound from a carbonate compound and an ester compound. The asymmetric carbonate compound obtained by the present invention is a useful compound for a solvent, various kinds of organic synthetic reagent, perfume, and functional materials. For example, it is useful for an electrolyte or a solvent of an alkaline electrochemical battery which is rechargeable such as a lithium secondary battery, etc.

## Claims

1. A process for preparing an asymmetric carbonate compound which comprises allowing a symmetric carbonate compound represented by the formula (1): 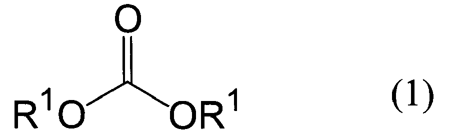 wherein R¹s are the same, and represent monovalent hydrocarbon groups which
may have a substituent(s), to react with an ester compound represented by the formula (2): 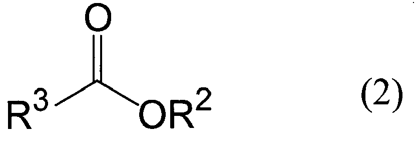 wherein
R² and R³ are each independently, and represent a monovalent hydrocarbon group which may have a substituent(s), where the hydrocarbon group of R² and R³ may be interrupted by a bonding group selected from the group consisting of an ether bond, a thioether bond, an amide bond, a carbonyl bond and an ester bond, or
R² and R³ are bonded together to form Z, and Z represents a divalent hydrocarbon group which may have a substituent(s), where the hydrocarbon group of Z may be interrupted by a bonding group selected from the group consisting of an ether bond, a thioether bond, an amide bond, a carbonyl bond and an ester bond, provided that R² is not the same as R¹ of the formula (1), and the number of ester bonds contained in the formula (2) is 2 or less in total,
in the presence of a hydrolase, to obtain an asymmetric carbonate compound represented by the formula (3): 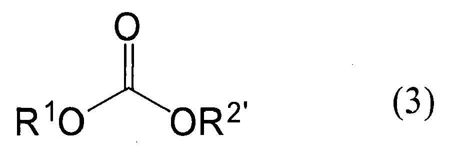 wherein
R¹ is as defined above, and
R^{2'} is R²,
provided that when R² and R³ form Z by bonding to each other, then R² is -Z-C(=O)-O-R¹.

2. The process for preparing an asymmetric carbonate compound according to Claim 1, wherein the symmetric carbonate compound represented by the formula (1) and an ester compound represented by the formula (2a): 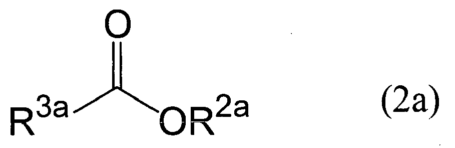 wherein
R^{2a} and R^{3a} are each independently, and represent a monovalent hydrocarbon group which may have a substituent(s), where the hydrocarbon group of R^{2a} and
R^{3a} may be interrupted by a bonding group selected from the group consisting of an ether bond, a thioether bond, an amide bond, a carbonyl bond and an ester bond,
provided that R^{2a} is not the same as R¹ of the formula (1), and the number of ester bonds contained in the formula (2a) is 2 or less in total,
are reacted in the presence of a hydrolase, to obtain an asymmetric carbonate compound represented by the formula (3a): 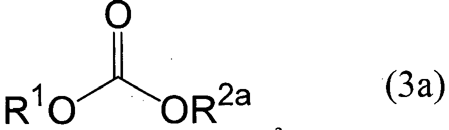 wherein R¹ is as defined in claim 1 and R^{2a} are the same as defined above.

3. The process for preparing an asymmetric carbonate compound according to Claim 2, wherein the reaction of the symmetric carbonate compound represented by the formula (1) and the ester compound represented by the formula (2a) is carried out while removing a transesterificated compound represented by the formula (4a): 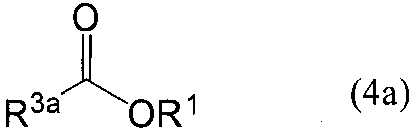 wherein R¹ and R^{3a} are as defined in Claim 2,
which is by-produced by the reaction.

4. The process for preparing an asymmetric carbonate compound according to Claim 3, wherein distillation of the transesterificated compound is carried out at a reaction pressure of 0.13 kPa or higher and less than 101.3 kPa.

5. The process for preparing an asymmetric carbonate compound according to any one of Claims 2 to 4, wherein R¹ and R^{2a} are different from each other, and each represents a linear alkyl group having 1 to 6 carbon atoms, a branched alkyl group having 3 to 6 carbon atoms or a cycloalkyl group having 3 to 6 carbon atoms.

6. The process for preparing an asymmetric carbonate compound according to any one of Claims 2 to 5, wherein R¹ is methyl, ethyl, n-propyl, isopropyl, n-butyl, s-butyl, isobutyl, t-butyl or benzyl.

7. The process for preparing an asymmetric carbonate compound according to any one of Claims 2 to 6, wherein R¹ is methyl or ethyl.

8. The process for preparing an asymmetric carbonate compound according to Claim 1, wherein the symmetric carbonate compound represented by the formula (1) and an ester compound represented by the formula (5a): 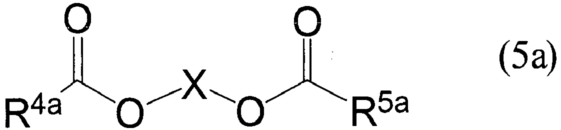 wherein
R^{4a} and R^{5a} are each independently, and represent a monovalent hydrocarbon group which may have a substituent(s),
X represents a divalent hydrocarbon group which may have a substituent(s) where the hydrocarbon group may be interrupted by a bonding group selected from the group consisting of an ether bond, a thioether bond, an amide bond and a carbonyl bond,
are reacted in the presence of a hydrolase, to obtain an asymmetric carbonate compound represented by the formula (6a): 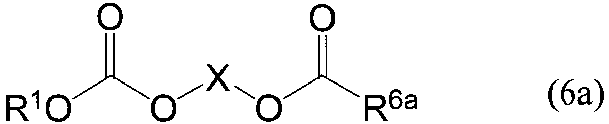 wherein
R^{6a} is R^{4a} or R^{5a} and
R¹ is as defined in claim 1, and X, R^{4a} and R^{5a} are as defined above.

9. The process for preparing an asymmetric carbonate compound according to Claim 8, wherein R^{4a} and R^{5a} are each independently, and represent a linear alkyl group having 1 to 6 carbon atoms, a branched alkyl group having 3 to 6 carbon atoms or a cycloalkyl group having 3 to 6 carbon atoms, and
X is an alkylene group having 1 to 6 carbon atoms or an alkenylene group having 1 to 6 carbon atoms, or a linear alkylene group having 2 to 6 carbon atoms which is interrupted by an ether bond.

10. The process for preparing an asymmetric carbonate compound according to Claim 1, wherein the symmetric carbonate compound represented by the formula (1) and an ester compound represented by the formula (5a): 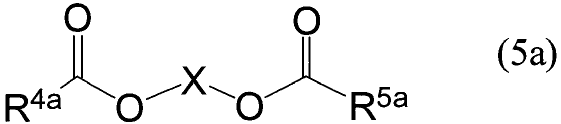 wherein
R^{4a} and R^{5a} are each independently, and represent a monovalent hydrocarbon group which may have a substituent(s),
X represents a divalent hydrocarbon group which may have a substituent(s), where the hydrocarbon group may be interrupted by a bonding group selected from the group consisting of an ether bond, a thioether bond, an amide bond and a carbonyl bond,
are reacted in the presence of a hydrolase, to obtain an asymmetric carbonate compound represented by the formula (7a): 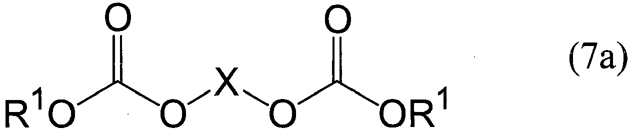 wherein R¹ and X are the same as defined above.

11. The process for preparing an asymmetric carbonate compound according to Claim 1, wherein the symmetric carbonate compound represented by the formula (1) and an ester compound represented by the formula (8a): 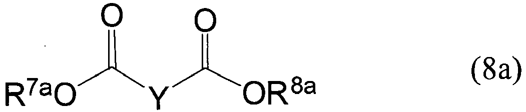 wherein
R^{7a} and R^{8a} are each independently, and represent a monovalent hydrocarbon group which may have a substituent(s), provided that each of R^{7a} and R^{8a} are not the same as R¹ in formula (1),
Y represents a divalent hydrocarbon group which may have a substituent(s) where, the hydrocarbon group may be interrupted by a bonding group selected from the group consisting of an ether bond, a thioether bond, an amide bond and a carbonyl bond,
are reacted in the presence of a hydrolase, to obtain an asymmetric carbonate compound represented by the formula (9a): 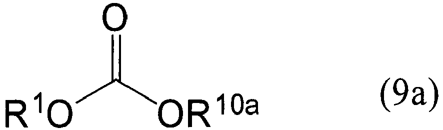 wherein
R^{10a} is R^{7a} or R^{8a}, and
R¹ is as defined in claim 1 and R^{7a} and R^{8a} are as defined above.

12. The process for preparing an asymmetric carbonate compound according to Claim 1, wherein the symmetric carbonate compound represented by the formula (1) and a cyclic ester compound represented by the formula (10): 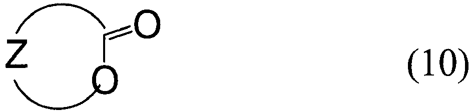 wherein Z represents a divalent hydrocarbon group which may have a substituent(s) where the hydrocarbon group may be interrupted by a bonding group selected from the group consisting of an ether bond, a thioether bond, an amide bond, a carbonyl bond and an ester bond, provided that the number of ester bonds contained in the formula (10) is 2 or less in total,
are reacted in the presence of a hydrolase, to obtain an asymmetric carbonate compound represented by the formula (11): 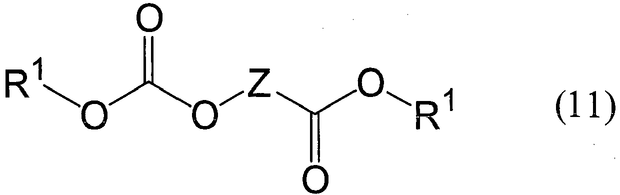 wherein Z is as defined above, and R¹s are as defined in claim land are the same.

13. The process for preparing an asymmetric carbonate compound according to Claim 12, wherein the cyclic ester compound represented by the formula (10) is δ-valerolactone, β-propiolactone or ε-caprolactone.

14. The process for preparing an asymmetric carbonate compound according to any one of Claims 1 to 13, wherein the hydrolase is at least one hydrolase selected from the group consisting of a protease, esterase and lipase.

15. The process for preparing an asymmetric carbonate compound according to Claim 14, wherein the hydrolase is a lipase.

16. The process for preparing an asymmetric carbonate compound according to Claim 15, wherein the hydrolase is a lipase originated from *Candida Antarctica.*

17. The process for preparing an asymmetric carbonate compound according to any one of Claims 1 to 16, wherein an organic solvent is used.

18. The process for preparing an asymmetric carbonate compound according to Claim 17, wherein the organic solvent is at least one organic solvent selected from the group consisting of cyclohexane, toluene and t-butyl methyl ether.

19. The process for preparing an asymmetric carbonate compound according to any one of Claims 1 to 18, wherein an amount of the symmetric carbonate compound represented by the formula (1) is 0.1 to 100 mol based on 1 mol of the ester compound represented by the formula (2).

20. The process for preparing an asymmetric carbonate compound according to any one of Claims 1 to 19, wherein the reaction temperature is 0 to 130°C.

## Patentansprüche

1. Verfahren zur Herstellung einer asymmetrischen Carbonatverbindung, das das Reagierenlassen einer symmetrischen Carbonatverbindung der Formel (1): 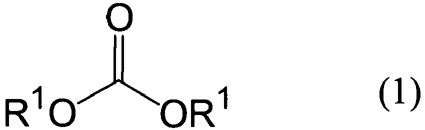 worin die R¹ gleich sind und monovalente Kohlenwasserstoffgruppen darstellen, die (einen) Substituenten aufweisen können,
mit einer Esterverbindung der Formel (2): 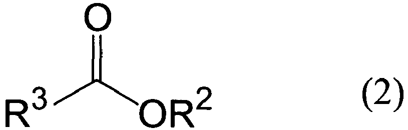 worin
R² und R³ unabhängig sind und eine monovalente Kohlenwasserstoffgruppe darstellen, die (einen) Substituenten aufweisen kann, wobei die Kohlenwasserstoffgruppe von R² und R³ durch eine Bindungsgruppe, ausgewählt aus der Gruppe bestehend aus einer Etherbindung, einer Thioetherbindung, einer Amidbindung, einer Carbonylbindung und einer Esterbindung, unterbrochen sein kann oder
R² und R³ miteinander verbunden sind, um Z zu bilden, und Z eine divalente Kohlenwasserstoffgruppe darstellt, die (einen) Substituenten aufweisen kann, wobei die Kohlenwasserstoffgruppe von Z durch eine Bindungsgruppe, ausgewählt aus der Gruppe bestehend aus einer Etherbindung, einer Thioetherbindung, einer Amidbindung, einer Carbonylbindung und einer Esterbindung, unterbrochen sein kann,
vorausgesetzt, dass R² nicht dasselbe wie R¹ in Formel (1) ist und die Zahl an Esterbindungen, die in Formel (2) enthalten sind, insgesamt 2 oder weniger ist,
in Gegenwart einer Hydrolase umfasst, um eine asymmetrische Carbonatverbindung der Formel (3) zu erhalten: 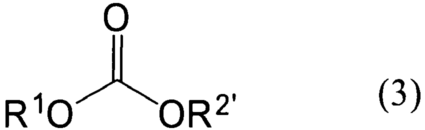 worin
R¹ wie oben definiert ist und
R^{2'} R² ist,
vorausgesetzt, dass, wenn R² und R³ durch Aneinanderbinden Z bilden, dann R²_{,} -Z-C(=O)-O-R¹ ist.

2. Verfahren zur Herstellung einer asymmetrischen Carbonatverbindung gemäss Anspruch 1, wobei die symmetrische Carbonatverbindung der Formel (1) und eine Esterverbindung der Formel (2a): 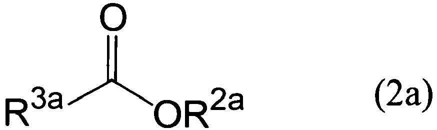 worin
R^{2a} und R^{3A} unabhängig sind und eine monovalente Kohlenwasserstoffgruppe darstellen, die (einen) Substituenten aufweisen kann, wobei die Kohlenwasserstoffgruppe von R^{2a} und R^{3A} durch eine Bindungsgruppe, ausgewählt aus der Gruppe bestehend aus einer Etherbindung, einer Thioetherbindung, einer Amidbindung, einer Carbonylbindung und einer Esterbindung, unterbrochen sein kann,
vorausgesetzt, dass R^{2a} nicht dasselbe wie R¹ in Formel (1) ist und die Zahl an Esterbindungen, die in Formel (2a) enthalten sind, insgesamt 2 oder weniger ist,
in Gegenwart einer Hydrolase umgesetzt werden, um eine asymmetrische Carbonatverbindung der Formel (3a) zu erhalten: 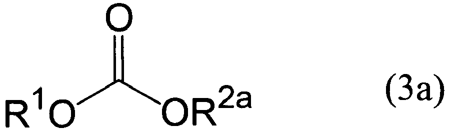 worin R¹ wie in Anspruch 1 definiert ist und R^{2a} so wie oben definiert ist.

3. Verfahren zur Herstellung einer asymmetrischen Carbonatverbindung gemäss Anspruch 2, wobei die Reaktion der symmetrischen Carbonatverbindung der Formel (1) und der Esterverbindung der Formel (2a) durchgeführt wird, während eine umgeesterte Verbindung der Formel (4a): 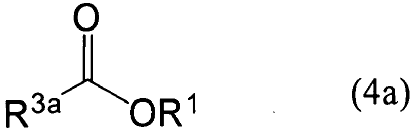 worin R¹ und R^{3A} wie in Anspruch 2 definiert sind, die ein Nebenprodukt der Reaktion ist, entfernt wird.

4. Verfahren zur Herstellung einer asymmetrischen Carbonatverbindung gemäss Anspruch 3, wobei eine Destillation der umgeesterten Verbindung bei einem Reaktionsdruck von 0,13 kPa oder höher und weniger als 101,3 kPa durchgeführt wird.

5. Verfahren zur Herstellung einer asymmetrischen Carbonatverbindung gemäss irgendeinem der Ansprüche 2 bis 4, worin R¹ und R^{2a} voneinander verschieden sind und eine geradkettige Alkylgruppe mit 1 bis 6 Kohlenstoffatomen, eine verzweigte Alkylgruppe mit 3 bis 6 Kohlenstoffatomen oder eine Cycloalkylgruppe mit 3 bis 6 Kohlenstoffatomen darstellen.

6. Verfahren zur Herstellung einer asymmetrischen Carbonatverbindung gemäss irgendeinem der Ansprüche 2 bis 5, worin R¹ Methyl, Ethyl, n-Propyl, Isopropyl, n-Butyl, s-Butyl, Isobutyl, t-Butyl oder Benzyl ist.

7. Verfahren zur Herstellung einer asymmetrischen Carbonatverbindung gemäss irgendeinem der Ansprüche 2 bis 6, worin R¹ Methyl oder Ethyl ist.

8. Verfahren zur Herstellung einer asymmetrischen Carbonatverbindung gemäss Anspruch 1, wobei die symmetrische Carbonatverbindung der Formel (1) und eine Esterverbindung der Formel (5a): 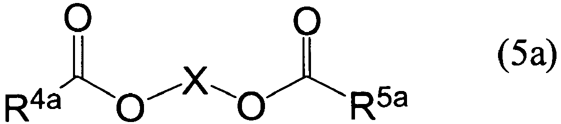 worin
R^{4a} und R^{5a} unabhängig sind und eine monovalente Kohlenwasserstoffgruppe darstellen, die (einen) Substituenten aufweisen kann,
X eine divalente Kohlenwasserstoffgruppe darstellt, die (einen) Substituenten aufweisen kann, wobei die Kohlenwasserstoffgruppe durch eine Bindungsgruppe, ausgewählt aus der Gruppe bestehend aus einer Etherbindung, einer Thioetherbindung, einer Amidbindung und einer Carbonylbindung, unterbrochen sein kann,
in Gegenwart einer Hydrolase umgesetzt werden, um eine asymmetrische Carbonatverbindung der Formel (6a) zu erhalten: 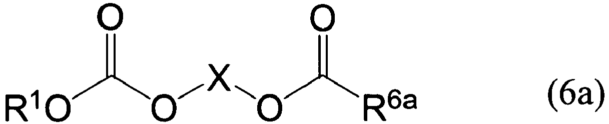 worin
R^{6a} R^{4a} oder R^{5a} ist und
R¹ wie in Anspruch 1 definiert ist und X, R^{4a} und R^{5a} wie oben definiert sind.

9. Verfahren zur Herstellung einer asymmetrischen Carbonatverbindung gemäss Anspruch 8, worin R^{4a} und R^{5a} unabhängig sind und eine geradkettige Alkylgruppe mit 1 bis 6 Kohlenstoffatomen, eine verzweigte Alkylgruppe mit 3 bis 6 Kohlenstoffatomen oder eine Cycloalkylgruppe mit 3 bis 6 Kohlenstoffatomen darstellen, und
X eine Alkylengruppe mit 1 bis 6 Kohlenstoffatomen oder eine Alkenylengruppe mit 1 bis 6 Kohlenstoffatomen oder eine geradkettige Alkylengruppe mit 2 bis 6 Kohlenstoffatomen ist, die durch einer Etherbindung unterbrochen ist.

10. Verfahren zur Herstellung einer asymmetrischen Carbonatverbindung gemäss Anspruch 1, wobei die symmetrische Carbonatverbindung der Formel (1) und eine Esterverbindung der Formel (5a): 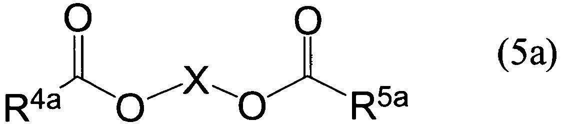 worin
R^{4a} und R^{5a} unabhängig sind und eine monovalente Kohlenwasserstoffgruppe darstellen, die (einen) Substituenten aufweisen kann,
X eine divalente Kohlenwasserstoffgruppe darstellt, die (einen) Substituenten aufweisen kann, wobei die Kohlenwasserstoffgruppe durch eine Bindungsgruppe, ausgewählt aus der Gruppe bestehend aus einer Etherbindung, einer Thioetherbindung, einer Amidbindung und einer Carbonylbindung, unterbrochen sein kann,
in Gegenwart einer Hydrolase umgesetzt werden, um eine asymmetrische Carbonatverbindung der Formel (7a) zu erhalten: 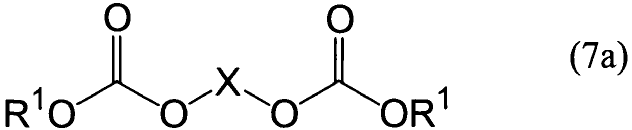 worin R¹ und X dieselben sind wie oben definiert.

11. Verfahren zur Herstellung einer asymmetrischen Carbonatverbindung gemäss Anspruch 1, wobei die symmetrische Carbonatverbindung der Formel (1) und eine Esterverbindung der Formel (8a): 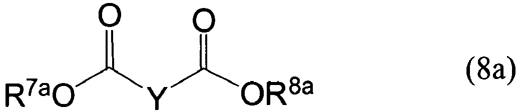 worin
R^{7a} und R^{8a} unabhängig sind und eine monovalente Kohlenwasserstoffgruppe darstellen, die (einen) Substituenten aufweisen kann, vorausgesetzt, dass keines von R^{7a} und R^{8a} dasselbe wie R¹ in Formel (1) ist,
Y eine divalente Kohlenwasserstoffgruppe darstellt, die (einen) Substituenten aufweisen kann, wobei die Kohlenwasserstoffgruppe durch eine Bindungsgruppe, ausgewählt aus der Gruppe bestehend aus einer Etherbindung, einer Thioetherbindung, einer Amidbindung und einer Carbonylbindung, unterbrochen sein kann,
in Gegenwart einer Hydrolase umgesetzt werden, um eine asymmetrische Carbonatverbindung der Formel (9a) zu erhalten: 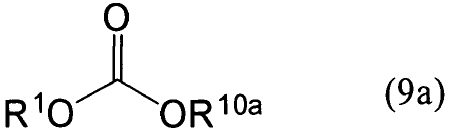 worin
R^{10a} R^{7a} oder R^{8a} ist, und
R¹ wie in Anspruch 1 definiert ist und R^{7a} und R^{8a} wie oben definiert sind.

12. Verfahren zur Herstellung einer asymmetrischen Carbonatverbindung gemäss Anspruch 1, wobei die symmetrische Carbonatverbindung der Formel (1) und eine cyclische Esterverbindung der Formel (10): 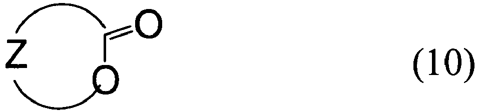 worin Z eine divalente Kohlenwasserstoffgruppe darstellt, die (einen) Substituenten aufweisen kann, wobei die Kohlenwasserstoffgruppe durch eine Bindungsgruppe, ausgewählt aus der Gruppe bestehend aus einer Etherbindung, einer Thioetherbindung, einer Amidbindung, einer Carbonylbindung und einer Esterbindung, unterbrochen sein kann, vorausgesetzt, dass die Zahl an Esterbindungen, die in Formel (10) enthalten sind, insgesamt 2 oder weniger ist,
in Gegenwart einer Hydrolase umgesetzt werden, um eine asymmetrische Carbonatverbindung der Formel (11) zu erhalten: 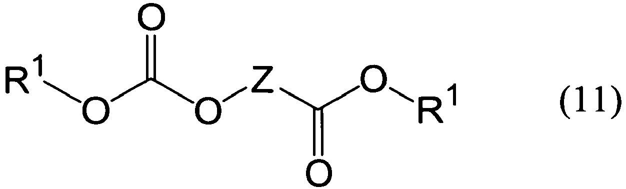 worin Z wie oben definiert ist und die R¹ so wie in Anspruch 1 definiert sind und dieselben sind.

13. Verfahren zur Herstellung einer asymmetrischen Carbonatverbindung gemäss Anspruch 12, wobei die cyclische Esterverbindung der Formel (10) δ-Valerolacton, β-Propiolacton oder ε-Caprolacton ist.

14. Verfahren zur Herstellung einer asymmetrischen Carbonatverbindung gemäss irgendeinem der Ansprüche 1 bis 13, wobei die Hydrolase zumindest eine Hydrolase, ausgewählt aus der Gruppe bestehend aus einer Protease, Esterase und Lipase, ist.

15. Verfahren zur Herstellung einer asymmetrischen Carbonatverbindung gemäss Anspruch 14, wobei die Hydrolase eine Lipase ist.

16. Verfahren zur Herstellung einer asymmetrischen Carbonatverbindung gemäss Anspruch 15, wobei die Hydrolase eine Lipase ist, die von Candida Antarctica stammt.

17. Verfahren zur Herstellung einer asymmetrischen Carbonatverbindung gemäss irgendeinem der Ansprüche 1 bis 16, wobei ein organisches Lösungsmittel verwendet wird.

18. Verfahren zur Herstellung einer asymmetrischen Carbonatverbindung gemäss Anspruch 17, wobei das organische Lösungsmittel zumindest ein organisches Lösungsmittel, ausgewählt aus der Gruppe bestehend aus Cyclohexan, Toluol und t-Butylmethylether, ist.

19. Verfahren zur Herstellung einer asymmetrischen Carbonatverbindung gemäss irgendeinem der Ansprüche 1 bis 18, wobei die Menge der symmetrischen Carbonatverbindung der Formel (1) 0,1 bis 100 mol, bezogen auf 1 mol der Esterverbindung der Formel (2), beträgt.

20. Verfahren zur Herstellung einer asymmetrischen Carbonatverbindung gemäss irgendeinem der Ansprüche 1 bis 19, wobei die Reaktionstemperatur 0 bis 130°C beträgt.

## Revendications

1. Procédé de préparation d'un composé de carbonate asymétrique qui comprend laisser un composé de carbonate symétrique représenté par la formule (1) : 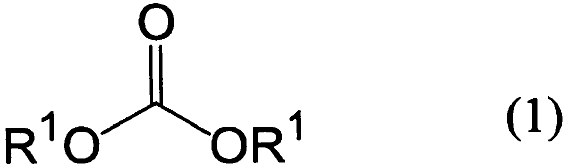 dans laquelle les R¹s sont les mêmes, et représentent des groupes hydrocarbonés monovalents qui peuvent avoir un substituant(s),
réagir avec un composé d'ester représenté par la formule (2) : 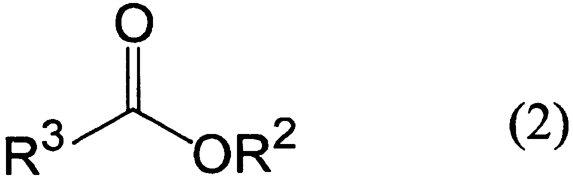 dans laquelle
R² et R³ sont chacun indépendamment, et représentent un groupe hydrocarboné monovalent qui peut avoir un substituant(s), où le groupe hydrocarboné de R² et
R³ peut être interrompu par un groupe de liaison sélectionné dans le groupe constitué d'une liaison éther, d'une liaison thioéther, d'une liaison amide, d'une liaison carbonyle et d'une liaison ester, ou R² et R³ sont liés ensemble pour former Z, et Z représente un groupe hydrocarboné divalent qui peut avoir un substituant(s), où le groupe hydrocarboné de Z peut être interrompu par un groupe de liaison sélectionné dans le groupe constitué d'une liaison éther, d'une liaison thioéther, d'une liaison amide, d'une liaison carbonyle et d'une liaison ester, sous réserve que R² ne soit pas le même que R¹ de la formule (1), et le nombre de liaisons ester contenues dans la formule (2) est 2 ou moins au total,
en présence d'une hydrolase, pour obtenir un composé de carbonate asymétrique représenté par la formule (3) : 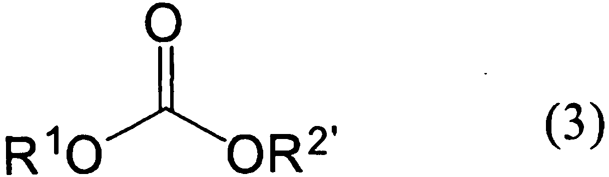 dans laquelle
R¹ est tel que défini ci-dessus, et
R^{2'} est R²,
sous réserve que lorsque R² et R³ forment Z par liaison l'un à l'autre, alors R^{2'} est -Z-C(=O)-O-R¹.

2. Procédé de préparation d'un composé de carbonate asymétrique selon la revendication 1, dans lequel le composé de carbonate symétrique représenté par la formule (1) et un composé d'ester représenté par la formule (2a) : 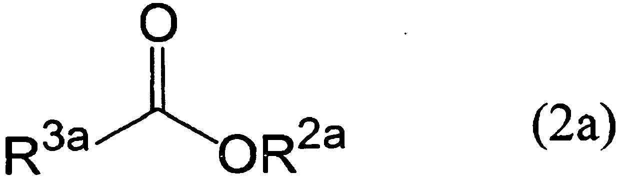 dans laquelle
R^{2a} et R^{3a} sont chacun indépendamment, et représentent un groupe hydrocarboné monovalent qui peut avoir un substituant(s), où le groupe hydrocarboné de R^{2a} et
R^{3a} peut être interrompu par un groupe de liaison sélectionné dans le groupe constitué d'une liaison éther, d'une liaison thioéther, d'une liaison amide, d'une liaison carbonyle et d'une liaison ester,
sous réserve que R^{2a} ne soit pas le même que R¹ de la formule (1), et le nombre de liaisons ester contenues dans la formule (2a) est 2 ou moins au total,
sont mis à réagir en présence d'une hydrolase, pour obtenir un composé de carbonate asymétrique représenté par la formule (3a) : 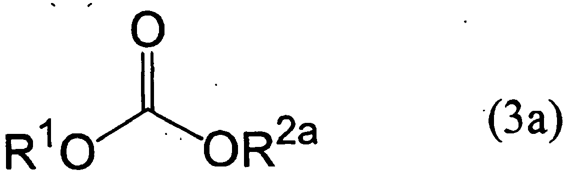 dans laquelle R¹ est tel que défini dans la revendication 1 et R^{2a} sont les mêmes que définis ci-dessus.

3. Procédé de préparation d'un composé de carbonate asymétrique selon la revendication 2, dans lequel la réaction du composé de carbonate symétrique représenté par la formule (1) et du composé d'ester représenté par la formule (2a) est réalisée en même temps que le retrait d'un composé transestérifié représenté par la formule (4a) : 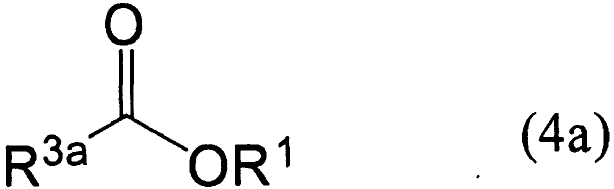 dans laquelle R¹ et R^{3a} sont tels que définis dans la revendication 2, qui est sous-produit par la réaction.

4. Procédé de préparation d'un composé de carbonate asymétrique selon la revendication 3, dans lequel une distillation du composé transestérifié est réalisée à une pression de réaction de 0,13 kPa ou plus et de moins de 101,3 kPa.

5. Procédé de préparation d'un composé de carbonate asymétrique selon l'une quelconque des revendications 2 à 4, dans lequel R¹ et R^{2a} sont différents l'un de l'autre, et chacun représente un groupe alkyle linéaire ayant 1 à 6 atomes de carbone, un groupe alkyle ramifié ayant 3 à 6 atomes de carbone ou un groupe cycloalkyle ayant 3 à 6 atomes de carbone.

6. Procédé de préparation d'un composé de carbonate asymétrique selon l'une quelconque des revendications 2 à 5, dans lequel R¹ est un méthyle, un éthyle, un n-propyle, un isopropyle, un n-butyle, un s-butyle, un isobutyle, un t-butyle ou un benzyle.

7. Procédé de préparation d'un composé de carbonate asymétrique selon l'une quelconque des revendications 2 à 6, dans lequel R¹ est un méthyle ou un éthyle.

8. Procédé de préparation d'un composé de carbonate asymétrique selon la revendication 1, dans lequel le composé de carbonate symétrique représenté par la formule (1) et un composé d'ester représenté par la formule (5a) : 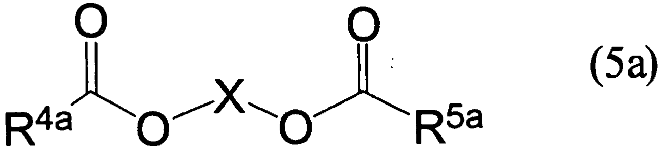 dans laquelle
R^{4a} et R^{5a} sont chacun indépendamment, et représentent un groupe hydrocarboné monovalent qui peut avoir un substituant(s),
X représente un groupe hydrocarboné divalent qui peut avoir un substituant(s) où le groupe hydrocarboné peut être interrompu par un groupe de liaison sélectionné dans le groupe constitué d'une liaison éther, d'une liaison thioéther, d'une liaison amide et d'une liaison carbonyle,
sont mis à réagir en présence d'une hydrolase, pour obtenir un composé de carbonate asymétrique représenté par la formule (6a) : 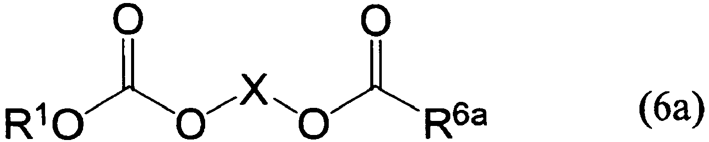 dans laquelle
R^{6a} est R^{4a} ou R^{5a}, et
R¹ est tel que défini dans la revendication 1, et X, R^{4a} et R^{5a} sont tels que définis ci-dessus.

9. Procédé de préparation d'un composé de carbonate asymétrique selon la revendication 8, dans lequel R^{4a} et R^{5a} sont chacun indépendamment, et représentent un groupe alkyle linéaire ayant 1 à 6 atomes de carbone, un groupe alkyle ramifié ayant 3 à 6 atomes de carbone ou un groupe cycloalkyle ayant 3 à 6 atomes de carbone, et X est un groupe alkylène ayant 1 à 6 atomes de carbone ou un groupe alcénylène ayant 1 à 6 atomes de carbone, ou un groupe alkylène linéaire ayant 2 à 6 atomes de carbone qui est interrompu par une liaison éther.

10. Procédé de préparation d'un composé de carbonate asymétrique selon la revendication 1, dans lequel le composé de carbonate symétrique représenté par la formule (1) et un composé d'ester représenté par la formule (5a) : 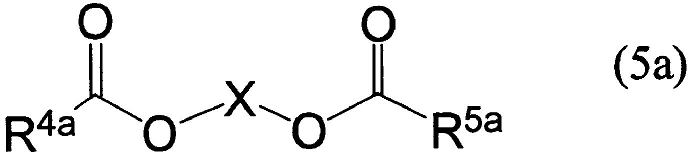 dans laquelle
R^{4a} et R^{5a} sont chacun indépendamment, et représentent un groupe hydrocarboné monovalent qui peut avoir un substituant(s),
X représente un groupe hydrocarboné divalent qui peut avoir un substituant(s), où le groupe hydrocarboné peut être interrompu par un groupe de liaison sélectionné dans le groupe constitué d'une liaison éther, d'une liaison thioéther, d'une liaison amide et d'une liaison carbonyle,
sont mis à réagir en présence d'une hydrolase, pour obtenir un composé de carbonate asymétrique représenté par la formule (7a) : 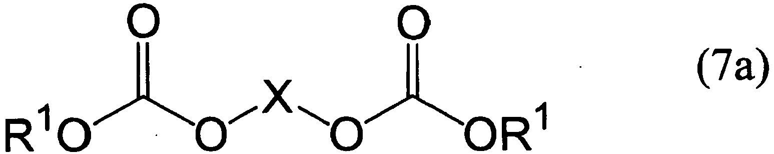 dans laquelle R¹ et X sont les mêmes que définis ci-dessus.

11. Procédé de préparation d'un composé de carbonate asymétrique selon la revendication 1, dans lequel le composé de carbonate symétrique représenté par la formule (1)
et un composé d'ester représenté par la formule (8a) : 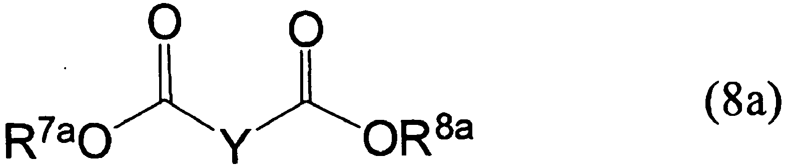 dans laquelle
R^{7a} et R^{8a} sont chacun indépendamment, et représentent un groupe hydrocarboné monovalent qui peut avoir un substituant(s), sous réserve que chacun de R^{7a} et R^{8a} ne soient pas le même que R¹ dans la formule (1),
Y représente un groupe hydrocarboné divalent qui peut avoir un substituant(s), où le groupe hydrocarboné peut être interrompu par un groupe de liaison sélectionné dans le groupe constitué d'une liaison éther, d'une liaison thioéther, d'une liaison amide et d'une liaison carbonyle,
sont mis à réagir en présence d'une hydrolase, pour obtenir un composé de carbonate asymétrique représenté par la formule (9a) : 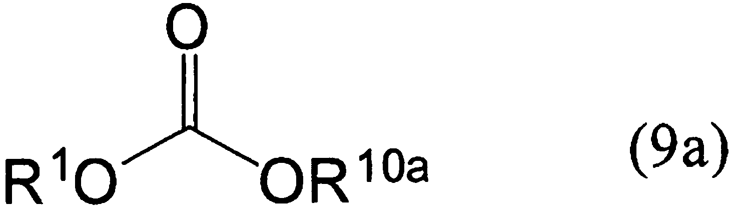 dans laquelle
R^{10a} est R^{7a} ou R^{8a}, et
R¹ est tel que défini dans la revendication 1 et R^{4a} et R^{8a} sont tels que définis ci-dessus.

12. Procédé de préparation d'un composé de carbonate asymétrique selon la revendication 1, dans lequel le composé de carbonate symétrique représenté par la formule (1)
et un composé d'ester cyclique représenté par la formule (10) : 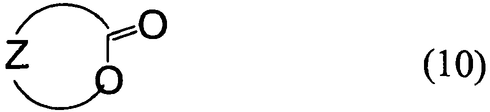 dans laquelle Z représente un groupe hydrocarboné divalent qui peut avoir un substituant(s) où le groupe hydrocarboné peut être interrompu par un groupe de liaison sélectionné dans le groupe constitué d'une liaison éther, d'une liaison thioéther, d'une liaison amide, d'une liaison carbonyle et une liaison ester, sous réserve que le nombre de liaisons ester contenues dans la formule (10) soit 2 ou moins au total,
sont mis à réagir en présence d'une hydrolase, pour obtenir un composé de carbonate asymétrique représenté par la formule (11) : 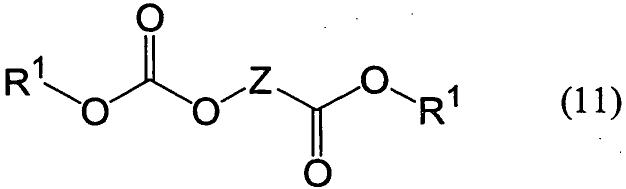 dans laquelle Z est tel que défini ci-dessus, et les R¹ sont tels que définis dans la revendication 1 et sont les mêmes.

13. Procédé de préparation d'un composé de carbonate asymétrique selon la revendication 12, dans lequel le composé d'ester cyclique représenté par la formule (10) est la δ-valérolactone, la β-propiolactone ou la ε-caprolactone.

14. Procédé de préparation d'un composé de carbonate asymétrique selon l'une quelconque des revendications 1 à 13, dans lequel l'hydrolase est au moins une hydrolase sélectionnée dans le groupe constitué d'une protéase, d'une estérase et d'une lipase.

15. Procédé de préparation d'un composé de carbonate asymétrique selon la revendication 14, dans lequel l'hydrolase est une lipase.

16. Procédé de préparation de composé de carbonate asymétrique selon la revendication 15, dans lequel l'hydrolase est une lipase provenant de *Candida Antarctica.*

17. Procédé de préparation d'un composé de carbonate asymétrique selon l'une quelconque des revendications 1 à 16, dans lequel un solvant organique est utilisé.

18. Procédé de préparation d'un composé de carbonate asymétrique selon la revendication 17, dans lequel le solvant organique est au moins un solvant organique sélectionné dans le groupe constitué du cyclohexane, du toluène et de l'éther méthylique de t-butyle.

19. Procédé de préparation d'un composé de carbonate asymétrique selon l'une quelconque des revendications 1 à 18, dans lequel une quantité du composé de carbonate asymétrique représenté par la formule (1) est de 0,1 à100 mol sur la base de 1 mol du composé d'ester représenté par la formule (2).

20. Procédé de préparation d'un composé de carbonate asymétrique selon l'une quelconque des revendications 1 à 19, dans lequel la température de réaction est 0 à 130°C.
